# EUROPÄISCHE PATENTSCHRIFT

(11) **EP 1 758 852 B1**
(45) Veröffentlichungstag und Bekanntmachung des Hinweises auf die Patenterteilung: **02.09.2009**
(21) Anmeldenummer: 05747867.9
(22) Anmeldetag: 07.06.2005
(51) Int. Cl.: C07C 317/32, C07D 317/58, A61K 31/145, A61P 25/06, A61P 25/36

(54) **SUBSTITUIERTE CYCLOPENTEN-VERBINDUNGEN**
SUBSTITUTED CYCLOPENTENE COMPOUNDS
COMPOSES CYCLOPENTENE SUBSTITUES

(30) Priorität: 09.06.2004 DE 102004027912; 19.11.2004 US 993545
(43) Veröffentlichungstag der Anmeldung: 07.03.2007
(73) Patentinhaber: Grünenthal GmbH, 52078 Aachen (DE)
(72) Erfinder: ZIMMER, Oswald, 52146 Würselen (DE); HAURAND, Michael, 52078 Aachen (DE); SCHIENE, Klaus, 40227 Düsseldorf (DE); GILLEN, Clemens, 52159 Roetgen (DE); SCHNEIDER, Johannes, 52223 Stolberg (DE); BAHRENBERG, Gregor, 52078 Aachen (DE)
(74) Vertreter: Brosch, Oliver
(86) Internationale Anmeldenummer: PCT/EP2005/006092
(87) Internationale Veröffentlichungsnummer: WO 2005/121078

(56) Entgegenhaltungen:
- WO-A-01/56382
- WO-A-97/28121
- WO-A-98/56779
- WO-A-20/04002420
- US-A- 5 700 816

## Beschreibung

Die vorliegende Erfindung betrifft substituierte Cyclopenten-Verbindungen der allgemeinen Formel I, Verfahren zu ihrer Herstellung, Arzneimittel enthaltend diese Verbindungen sowie deren Verwendung zur Herstellung von Arzneimitteln.

Das aus 37 Aminosäuren bestehende Neuropeptid α-CGRP (α-Calcitonin Gene-Related Peptide) entsteht durch alternatives Spleißen des Calcitonin-Gens. Darüber hinaus ist ein weiteres CGRP, das β-CGRP, bekannt, das eine hohe Sequenzanalogie zu dem α-CGRP aufweist, jedoch von einem anderen Gen transkribiert wird.

Diese Neuropeptide werden in den Nervenenden des zentralen und peripheren Nervensystems gespeichert bzw. freigesetzt und entfalten ihre physiologische Wirkung über die Bindung an spezifische G-Protein gekoppelte Rezeptoren, die sogenannten CGRP-Rezeptoren. Diese CGRP-Rezeptoren, von denen verschiedene Subtypen wie CGRP1 und CGRP2 bekannt sind, sind an der Oberfläche von Zellen unterschiedlicher Gewebe, wie zum Beispiel Muskelzellen, glandulären Zellen, Epithelzellen oder neuronalen Zellen, lokalisiert.

Entsprechend der breiten Verteilung von CGRP-Rezeptoren in Geweben unterschiedlicher Spezialisierung, ist das CGRP-Peptid-Rezeptor-System für eine Vielzahl von physiologischen und pathophysiologischen Prozessen, wie z.B. Prozessen des cardiovaskulären Systems, des zentralen und/oder peripheren Nervensystems, des respiratorischen Systems oder des endokrinen Systems, von zentraler Bedeutung. So wird zum Beispiel in Patienten mit akuter Migräne sowie in Patienten mit Clusterkopfschmerz eine gegenüber einem gesunden Vergleichskollektliv erhöhte Konzentration des Neuropeptids CGRP im Plasma gefunden.

Die Regulation von CGRP-Rezeptoren, insbesondere die Inhibierung von CGRP-Rezeptoren mit Hilfe eines Rezeptor-spezifischen Antagonisten, eröffnet daher einen Ansatzpunkt für die erfolgreiche Therapie von Störungen und Erkrankungen, die mit dem CGRP-Ligand-Rezeptor-System in Zusammenhang stehen.

Die Aufgabe der vorliegenden Erfindung bestand daher darin, neue Verbindungen zur Verfügung zu stellen, die sich insbesondere als pharmazeutische Wirkstoffe in Arzneimitteln eignen. Vorzugsweise sollen sich diese Verbindungen zur CGRP-Rezeptor-Regulation, insbesondere als Antagonisten zur Inhibierung von CGRP-Rezeptoren eignen. Ebenfalls bevorzugt sollen sich diese Verbindungen zur Behandlung und/oder Prophylaxe von Störungen und/oder Krankheiten, die zumindest teilweise durch den CGRP-Rezeptor, vorzugsweise durch den CGRP-1-Rezeptor, vermittelt werden, zur Prophylaxe und/oder Behandlung von Schmerzen, vorzugsweise von akuten Schmerzen, chronischen Schmerzen, chronisch entzündlichen Schmerzen und/oder viszeralen Schmerzen, Clusterkopfschmerzen, neurovaskulären Störungen, Migräne, vorzugsweise von Migräne mit Aura, Migräne ohne Aura, einfacher Migräne, klassischer Migräne oder komplizierter Migräne, Entzündungen, vorzugsweise Lungenentzündungen, Asthma, Arthritis, Hypertonie, Hypotonie, Tachykardie, nicht-insulinabhängigem Diabetes Mellitus, cardiovaskulären Erkrankungen, Hauterkrankungen und/oder Hautschäden, vorzugsweise von thermisch und/oder strahlenbedingten Hautschäden, besonders bevorzugt von durch Sonnenbrand hervorgerufenen Hautschäden, allergischer Rhinitis, Erkrankungen, die mit einer überschießenden Gefäßerweiterung einhergehen, vorzugsweise Schock oder Sepsis, menopausalen Hitzewallungen oder Opioidtoleranz, vorzugsweise Morphintoleranz, eignen.

Überraschenderweise wurde nun gefunden, daß die erfindungsgemäßen substituierten Cyclopenten-Verbindungen der nachstehenden allgemeinen Formel I eine hohe Affinität für den CGRP-Rezeptor aufweisen und als Antagonisten insbesondere zur Inhibierung des CGRP-Rezeptors, vorzugsweise des CGRP1-Rezeptors geeignet sind.

Im Stand der Technik sind cyclopentenyl-substituierte Verbindungen (WO 2004/002420) sowie substituierte Verbindungen bekannt, die eine Affinität zum CGRP-Rezeptor (US 5,700,816) oder zum COX-2 (Cyclooxygenase-2-Rezeptor) aufweisen (WO 97/28121). Ferner sind Verbindungen bekannt, die sich zur Behandlung von Diabetes eignen (WO 01/56382).

Ein Gegenstand der vorliegenden Erfindung sind daher substituierte Cyclopenten-Verbindungen der allgemeinen Formel I, 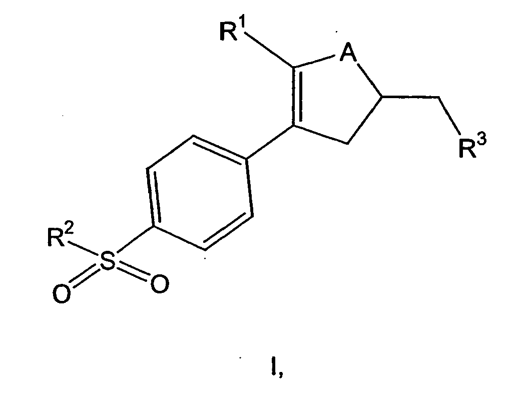 worin
R¹ für einen ggf. wenigstens einfach substituierten Phenyl-Rest steht, der mit einem ggf. wenigstens einfach substituierten, gesättigten, ungesättigten oder aromatischen, ggf. wenigstens ein Heteroatom als Ringglied aufweisenden monozyklischen Ringsystem kondensiert (anneliert) sein kann,
R² für einen geradkettigen oder verzweigten Alkyl-Rest oder für eine -NH₂-Gruppe steht,
R³ für eine -NR⁴R⁵-Gruppe oder eine -NR⁶R⁷-Gruppe steht,
R⁴ und R⁵, gleich oder verschieden, jeweils für Wasserstoff oder einen geradkettigen oder verzweigten Alkyl-Rest stehen, mit der Maßgabe, daß nicht beide Reste R⁴ und R⁵ gleichzeitig Wasserstoff bedeuten,
R⁶ und R⁷ gemeinsam mit dem sie verbrückenden Stickstoffatom als Ringglied einen gesättigten, ggf. wenigstens einfach substituierten, ggf. wenigstens ein weiteres Heteroatom als Ringglied aufweisenden heterozyklischen Rest bilden,
A für eine C(=O)-Gruppe oder für eine C(H)(R⁸)-Gruppe steht,
R⁸ für eine OH-Gruppe oder für eine -O-(C=O)-R⁹ -Gruppe steht,
R⁹ für einen geradkettigen oder verzweigten Alkyl-Rest steht,
jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Unter einem monozyklischen Ringsystem werden im Sinne der vorliegenden Erfindung monozyklische Kohlenwasserstoffreste verstanden, die gesättigt, ungesättigt oder aromatisch sein können. Ggf. kann das monozyklische Ringsystem auch ein oder mehrere, beispielsweise 1, 2 oder 3, Heteroatome als Ringglieder aufweisen, wobei die Heteroatome gleich oder verschieden sein können und jeweils bevorzugt ausgewählt sind aus der Gruppe bestehend aus Sauerstoff, Stickstoff und Schwefel. Bevorzugt weist das monozyklische Ringsystem 5 oder 6 Ringglieder auf.

Sofern R¹ für einen wenigstens einfach, beispielsweise 1-, 2-, 3-, 4- oder 5-fach, substituierten Phenyl-Rest steht und/oder ein wenigstens einfach, beispielsweise 1-, 2-, 3-, 4- oder 5-fach, substituiertes, gesättigtes, ungesättigtes oder aromatisches, ggf. wenigstens ein Heteroatom als Ringglied enthaltendes monozyklisches Ringsystem aufweist, können die jeweiligen Substituenten, jeweils unabhängig voneinander, bevorzugt ausgewählt werden aus der Gruppe bestehend aus Halogen, OH, CF₃, CF₂H, CFH₂, einem linearen oder verzeigten C₁₋₄-Alkyl-Rest, -SO₂R^{A} worin R^{A} für eine NH₂-Gruppe oder einen linearen oder verzweigten C₁₋₄-Alkyl-Rest steht, und OR^{B}, worin R^{B} für einen linearen oder verzweigten C₁₋₄-Alkyl-Rest, besonders bevorzugt aus der Gruppe bestehend aus F, Cl, Br, Methyl, Ethyl, n-Propyl, iso-Propyl, n-Butyl, sec-Butyl, tert-Butyl, OH, CF₃, OCH₃, OCH₂CH₃, SO₂NH₂ und SO₂CH₃, ganz besonders bevorzugt aus der Gruppe bestehend aus F, Cl, Methyl, Ethyl, iso-Propyl, tert-Butyl, OH, CF₃, OCH₃, OCH₂CH₃, und SO₂CH₃.

Sofern die Reste R⁶ und R⁷ gemeinsam mit dem sie verbrückenden Stickstoffatom als Ringglied einen gesättigten, wenigstens einfach, beispielsweise 1-, 2-, 3-, 4- oder 5-fach, substituierten, ggf. wenigstens ein, beispielsweise 1, 2 oder 3, weitere(s) Heteroatom(e) als Ringglied(er) aufweisenden heterozyklischen Rest bilden, können die jeweiligen Substituenten, jeweils unabhängig voneinander, bevorzugt ausgewählt werden aus der Gruppe bestehend aus Halogen, OH, CF₃, CF₂H, CFH₂, einem linearen oder verzeigten C₁₋₄-Alkyl-Rest, -SO₂R^{A} worin R^{A} für eine NH₂-Gruppe oder einen linearen oder verzweigten C₁₋₄-Alkyl-Rest steht, und OR^{B}, worin R^{B} für einen linearen oder verzweigten C₁₋₄-Alkyl-Rest, besonders bevorzugt aus der Gruppe bestehend aus F, Cl, Br, Methyl, Ethyl, n-Propyl, iso-Propyl, n-Butyl, sec-Butyl, tert-Butyl, OH, CF₃, OCH₃, OCH₂CH₃, SO₂NH₂ und SO₂CH₃, ganz besonders bevorzugt aus der Gruppe bestehend aus F, Cl, Methyl, Ethyl, iso-Propyl, tert-Butyl, OH, CF₃, OCH₃, OCH₂CH₃, und SO₂CH₃. Sofern der heterozyklische Rest eines oder mehrere, beispielsweise 1, 2 oder 3, weitere Heteroatome aufweist, können diese bevorzugt ausgewählt werden aus der Gruppe bestehend aus Sauerstoff, Stickstoff und Schwefel. Besonders bevorzugt können als weitere Heteroatome Sauerstoff und/oder Stickstoff vorliegen.

Bevorzugt sind substituierte Cyclopenten-Verbindungen der allgemeinen Formel I, in denen R¹ für einen ggf. wenigstens einfach substituierten Phenyl-Rest steht, der mit einem ggf. wenigstens einfach substituierten, gesättigten, ungesättigten oder aromatischen, ggf. wenigstens ein Heteroatom als Ringglied aufweisenden, 5-oder 6-gliedrigen monozyklischen Ringsystem kondensiert sein kann, vorzugsweise für einen ggf. wenigstens einfach substituierten Phenyl-, Naphthyl- oder Benzo[1,3]-dioxol-Rest steht, und jeweils die übrigen Reste R²-R⁹ und A die vorstehend genannte Bedeutung haben, ggf. jeweils in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin bevorzugt sind substituierte Cyclopenten-Verbindungen der allgemeinen Formel I, worin R² für einen geradkettigen oder verzweigten C₁₋₆-Alkyl-Rest oder für eine -NH₂-Gruppe, vorzugsweise für einen geradkettigen oder verzweigten C₁₋₄-Alkyl-Rest oder für eine NH₂-Gruppe, besonders bevorzugt für einen Methyl-Rest, einen Ethyl-Rest oder für eine NH₂-Gruppe, ganz besonders bevorzugt für einen Methyl-Rest steht, und jeweils die übrigen Reste R¹, R³-R⁹ und A die vorstehend genannte Bedeutung haben, ggf. jeweils in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Bevorzugt sind auch solche substituierte Cyclopenten-Verbindungen der allgemeinen Formel I, in denen R³ für eine -NR⁴R⁵-Gruppe steht und jeweils die übrigen Reste R¹, R², R⁸, R⁹ und A die vorstehend genannte Bedeutung haben, ggf. jeweils in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls bevorzugt sind substituierte Cyclopenten-Verbindungen der allgemeinen Formel I, in denen R⁴ und R⁵, gleich oder verschieden, jeweils für Wasserstoff oder einen geradkettigen oder verzweigten C₁₋₆-Alkyl-Rest, vorzugsweise jeweils für Wasserstoff oder einen geradkettigen oder verzweigten C₁₋₄-Alkyl-Rest, besonders bevorzugt jeweils für Wasserstoff, einen Methyl-Rest oder einen Ethyl-Rest, ganz besonders bevorzugt beide gleichzeitig für einen Methyl-Rest stehen, und jeweils die übrigen Reste R¹ bis R³, R⁶-R⁹ und A die vorstehend genannte Bedeutung haben, ggf. jeweils in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin bevorzugt sind substituierte Cyclopenten-Verbindungen der allgemeinen Formel I, in denen R⁶ und R⁷ gemeinsam mit dem sie verbrückenden Stickstoffatom als Ringglied einen gesättigten, ggf. wenigstens einfach substituierten, ggf. wenigstens ein weiteres Heteroatom als Ringglied aufweisenden, 5- bis 8-gliedrigen heterozyklischen Rest, vorzugsweise einen ggf. wenigstens einfach substituierten Pyrrolidin-, Piperidin-, Hexamethylenimin- oder Morpholin-Ring bilden, und jeweils die übrigen Reste R¹-R⁵, R⁸, R⁹ und A die vorstehend genannte Bedeutung haben, ggf. jeweils in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Bevorzugt sind auch substituierte Cyclopenten-Verbindungen der allgemeinen Formel I, worin A für eine -C(=O)-Gruppe steht und jeweils die übrigen Reste R¹ bis R⁹ die vorstehend genannte Bedeutung haben, ggf. jeweils in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls bevorzugt sind substituierte Cyclopenten-Verbindungen der allgemeinen Formel I, in denen R⁹ für einen geradkettigen oder verzweigten C₁₋₆-Alkyl-Rest, vorzugsweise für einen geradkettigen oder verzweigten C₁₋₄-Alkyl-Rest , besonders bevorzugt für einen Methyl-Rest steht, und jeweils die Reste R¹ bis R⁸ und A die vorstehend genannte Bedeutung haben, jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Bevorzugt sind auch Verbindungen, in denen
R¹ für einen Phenyl-, Naphtyl- oder Benzo[1,3]dioxolyl-Rest steht, der jeweils unsubstituiert oder mit 1, 2, 3, 4 oder 5 Substituenten unabhängig voneinander ausgewählt aus der Gruppe bestehend aus -F; -Cl; -Br; -I; -CF₃; -OH; Methyl; Ethyl; n-Propyl; iso-Propyl; n-Butyl; sec-Butyl; iso-Butyl; tert-Butyl; Methoxy; Ethoxy; n-Propyloxy; iso-Propyloxy; n-Butyloxy; sec-Butyloxy; iso-Butyloxy; tert-Butyloxy; -SO₂-methyl; -SO₂-ethyl; -SO₂-n-propyl; -SO₂-iso-propyl; -SO₂-n-butyl; -SO₂-sec-butyl; -SO₂-iso-butyl; -SO₂-tert-butyl und -SO₂-NH₂ substituiert ist,
R² für eine Methyl-Gruppe oder für eine -NH₂-Gruppe steht,
R³ für eine -NR⁴R⁵-Gruppe steht,
R⁴ und R⁵ jeweils für eine Methyl-Gruppe stehen;
A für eine C(=O)-Gruppe oder für eine C(H)(R⁸)-Gruppe steht,
R⁸ für eine OH-Gruppe oder für eine -O-(C=O)-R⁹ -Gruppe steht,
R⁹ für eine Methyl- oder Ethyl-Gruppe steht;
jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls bevorzugt sind Verbindungen, in denen R¹ für einen Rest steht, der ausgewählt ist aus der Gruppe bestehend aus
2-Fluor-phenyl; 3-Fluor-phenyl; 4-Fluor-phenyl; 2-Chlor-phenyl; 3-Chlor-phenyl; 4-Chlor-phenyl; 2-Brom-phenyl; 3-Brom-phenyl; 4-Brom-phenyl; 3,5-Difluorphenyl; 3,5-Dichlor-phenyl; 3,5-Dibrom-phenyl; 2-Trifluormethyl-phenyl; 3-Trifluormethyl-phenyl; 4-Trifluormethyl-phenyl; 4-Fluor-3-Methyl-phenyl; o-Tolyl; m-Tolyl; p-Tolyl; 2-Ethyl-phenyl; 3-Ethyl-phenyl; 4-Ethyl-phenyl; 2-iso-Propyl-phenyl; 3-iso-Propyl-phenyl; 4-iso-Propyl-phenyl; 2-tert-Butyl-phenyl; 3-tert-Butyl-phenyl; 4-tert-Butyl-phenyl; 3,4-Dimethyl-phenyl; 2-Hydroxy-phenyl; 3-Hydroxy-phenyl; 4-Hydroxy-phenyl; 2-Methoxy-phenyl; 3-Methoxy-phenyl; 4-Methoxy-phenyl; 2-Ethoxy-phenyl; 3-Ethoxy-phenyl; 4-Ethoxy-phenyl; 2-Methansulfonyl-phenyl; 3-Methansulfonyl-phenyl; 4-Methansulfonyl-phenyl; Benzo[1,3}dioxo-5-yl; Naphth-1-yl und Naphth-2-yl.

Besonders bevorzugt sind substituierte Cyclopenten-Verbindungen der allgemeinen Formel I ausgewählt aus der Gruppe bestehend aus
2-(3,5-Difluor-phenyl)-5-dimethylaminomethyl-3-(4-methansulfonyl-phenyl)-cyclopent-2-enon,
5-Dimethylaminomethyl-3-(4-methansulfonyl-phenyl)-2-m-tolyl-cyclopent-2-enon,
5-Dimethylaminomethyl-2-(3-hydroxy-phenyl)-3-(4-methansulfonyl-phenyl)-cyclopent-2-enon,
5-Dimethylaminomethyl-2-(4-fluor-phenyl)-3-(4-methansulfonyl-phenyl)-cyclopent-2-enon,
5-Dimethylaminomethyl-2-(3-fluor-phenyl)-3-(4-methansulfonyl-phenyl)-cyclopent-2-enon,
2-(3-Chlor-phenyl)-5-dimethylaminomethyl-3-(4-methansulfonyl-phenyl)-cyclopent-2-enon,
5-Dimethylaminomethyl-3-(4-methansulfonyl-phenyl)-2-p-tolyl-cyclopent-2-enon,
5-Dimethylaminomethyl-2-(4-hydroxy-phenyl)-3-(4-methansulfonyl-phenyl)-cyclopent-2-enon,
5-Dimethylaminomethyl-3-(4-methansulfonyl-phenyl)-2-(3-trifluormethylphenyl)-cyclopent-2-enon,
5-Dimethylaminomethyl-3-(4-methansulfonyl-phenyl)-2-(4-methoxyphenyl)-cyclopent-2-enon,
2-Benzo[1,3]dioxol-5-y!-5-dimethylaminomethyl-3-(4-methansulfonyl-phenyl)-cyclopent-2-enon,
2-(3,5-Dichlor-phenyl)-5-dimethylaminomethyl-3-(4-methansulfonyl-phenyl)-cyclopent-2-enon,
5-Dimethylaminomethyl-3-(4-methansulfonyl-phenyl)-2-(3-methoxy-phenyl)-cyclopent-2-enon,
5-Dimethylaminomethyl-2-(4-fluor-3-methyl-phenyl)-3-(4-methansulfonyl-phenyl)-cyclopent-2-enon,
2-(4-tert-Butyl-phenyl)-5-dimethylaminomethyl-3-(4-methansulfonyl-phenyl)-cyclopent-2-enon,
5-Dimethylaminomethyl-2-(3-ethoxy-phenyl)-3-(4-methansulfonyl-phenyl)-cyclopent-2-enon,
5-Dimethylaminomethyl-2,3-bis-(4-methansulfonyl-phenyl)-cyclopent-2-enon,
5-Dimethylaminomethyl-3-(4-methansulfonyl-phenyl)-2-naphth-2-yl-cyclopent-2-enon,
5-Dimethylaminomethyl-2-(3,4-dimethyl-phenyl)-3-(4-methansulfonyl-phenyl)-cyclopent-2-enon,
5-Dimethylaminomethyl-2-(3-isopropyl-phenyl)-3-(4-methansulfonyl-phenyl)-cyclopent-2-enon,
2-(4-tert-Butyl-phenyl)-5-dimethylaminomethyl-3-(4-methansulfonyl-phenyl)-cyclopent-2-enol,
Essigsäure-2-(4-tert-Butyl-phenyl)-5-dimethylaminomethyl-3-(4-methansulfonyl-phenyl)-cyclopent-2-enylester,
5-Dimethylaminomethyl-3-(4-methansulfonyl-phenyl)-2m-tolyl-cyclopent-2-enol,
und jeweils entsprechenden Salzen, vorzugsweise Hydrochloriden, und jeweils entsprechenden Solvaten, vorzugsweise Hydraten.

Ein weiterer Gegenstand der vorliegenden Erfindung ist ein Verfahren zur Herstellung substituierter Cyclopenten-Verbindungen der vorstehend angegebenen allgemeinen Formel I, gemäß dem wenigstens eine Verbindung der allgemeinen Formel II 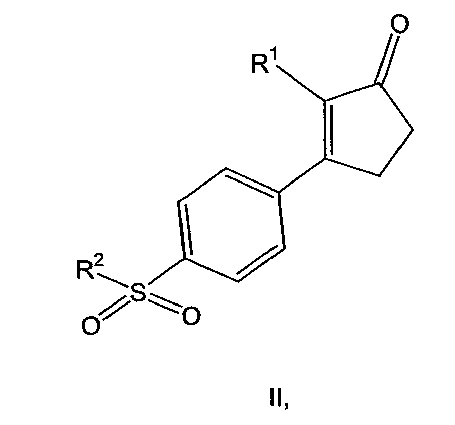 worin R¹ und R² die vorstehend genannte Bedeutung haben, durch Umsetzung mit Formaldehyd und/oder Paraformaldehyd und wenigstens einer substituierten Aminverbindung der allgemeinen Formel IIIa 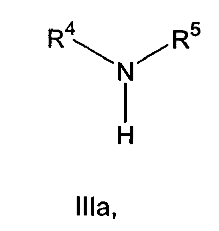 worin R⁴ und R⁵ die vorstehend genannte Bedeutung haben, oder wenigstens einer substituierten Aminverbindung der allgemeinen Formel IIIb, 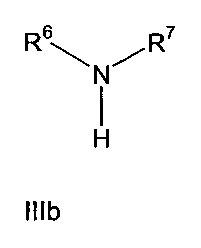 worin R⁶ und R⁷ die vorstehend genannte Bedeutung haben, oder durch Umsetzung mit wenigstens einer Methylenimmonium-Verbindung der allgemeinen Formel IVa 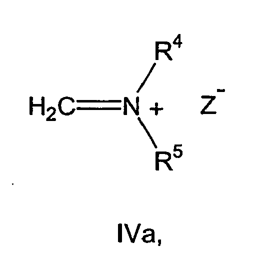 worin R⁴ und R⁵ die vorstehend genannte Bedeutung haben und Z für ein Chlor-, Brom- oder lodatom steht, oder mit wenigstens einer Methylenimmonium-Verbindung der allgemeinen Formel IVb, worin 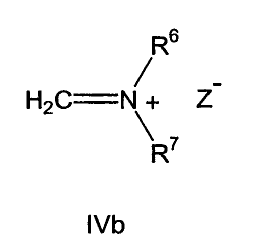 worin R⁶ und R⁷ die vorstehend genannte Bedeutung haben und Z für ein Chlor-, Brom- oder lodatom steht, in eine Verbindung der vorstehend angegebenen allgemeinen Formel I worin R¹ bis R⁷ die vorstehend genannte Bedeutung haben, und A für eine -(C=O)-Gruppe steht, übergeführt, ggf. gereinigt, ggf. isoliert, und ggf. durch Umsetzung mit wenigstens einem Reduktionsmittel in eine Verbindung der vorstehend angegebenen allgemeinen Formel I, worin R¹ bis R⁷ die vorstehend genannte Bedeutung haben, A für eine C(H)(R⁸)-Gruppe und R⁸ für eine Hydroxy-Gruppe steht, übergeführt, ggf. gereinigt, ggf. isoliert wird,
und diese ggf. durch Umsetzung mit einem Veresterungsreagenz in eine Verbindung der vorstehend angegebenen allgemeinen Formel I, worin R¹ bis R⁷ die vorstehend genannte Bedeutung haben, A für eine C(H)(R⁸)-Gruppe und R⁸ für eine -O(C=O)-R⁹-Gruppe steht, worin R⁹ die vorstehend angegebene Bedeutung hat, und diese ggf. gereinigt und ggf. isoliert wird.

Die Herstellung der Verbindungen der allgemeinen Formel II kann nach üblichen, dem Fachmann bekannten Methoden erfolgen, wie beispielsweise in D. Zhao et al., Tetrahedron 1999, 55, Seiten 6001-6018 und W. Cameron Black et al., J. Med. Chem., 1999, 42, Seiten 1274-1281 beschrieben. Die entsprechende Beschreibung wird hiermit als Referenz eingeführt und gilt als Teil der Offenbarung. Die Verbindungen IIIa, IIIb, IVa und IVb können ebenfalls nach üblichen, dem Fachmann bekannten Methoden oder kommerziell am Markt erhalten werden.

Die Umsetzung von Verbindungen der allgemeinen Formel II mit Formaldehyd und/oder Paraformaldehyd sowie mit Verbindungen der allgemeinen Formeln IIIa oder IIIb kann bevorzugt in einem polaren Reaktionsmedium wie Wasser, Alkoholen, Essigsäuren oder in einem Gemisch aus wenigstens zwei dieser Reaktionsmedien durchgeführt werden.

Die Umsetzung von Verbindungen der allgemeinen Formel II mit Verbindungen der allgemeinen Formeln IVa oder IVb kann bevorzugt in einem Reaktionsmedium auf Basis von Acetonitril und/oder Tetrahydrofuran durchgeführt werden.

Temperatur und Druck können während dieser vorstehend genannten Umsetzungen über einen weiten Bereich variieren. Vorzugsweise erfolgt die Umsetzung mit Verbindungen der allgemeinen Formel IIIa oder IIIb bei einer Temperatur von 10 °C bis zur Siedetemperatur des Reaktionsmediums, vorzugsweise von 20 °C bis zur Siedetemperatur des Reaktionsmediums. Die Umsetzung mit Verbindungen der allgemeinen Formel IVa und IVb erfolgt bevorzugt bei einer Temperatur von 10 bis 55 °C, besonders bevorzugt bei einer Temperatur von 20 bis 50 °C.

Die Reduktion von Verbindungen der allgemeinen Formel I, in denen A für eine - (C=O)-Gruppe steht zu Verbindungen der allgemeinen Formel I, in denen A für eine C(H)(R⁸)-Gruppe steht, kann nach üblichen, dem Fachmann bekannten Methoden mit üblichen, dem Fachmann bekannten Reduktionsmitteln und Reaktionsmedien erfolgen.

Der Fachmann versteht, daß das/die Reduktionsmittel so gewählt werden sollten, daß die Doppelbindung des Cyclopentenrings nicht angegriffen wird. Bevorzugt werden als Reduktionsmittel komplexe Borhydride und/oder komplexe Aluminiumhydride, besonders bevorzugt Natriumborhydrid eingesetzt.

Bevorzugte Reaktionsmedien für die Reduktion sind Alkohole, insbesondere Methanol, oder Gemische auf Basis von Alkoholen, insbesondere Methanol. Vorzugsweise wird die Reduktion bei einer Temperatur von 10 bis 60 °C, vorzugsweise 20 bis 50 °C, durchgeführt. Besonders bevorzugt erfolgt die Reduktion mit Natriumborhydrid in Methanol bei einer Temperatur von 20 bis 50 °C.

Die Veresterung von Verbindungen der allgemeinen Formel I, in denen A für eine C(H)(R⁸)-Gruppe und R⁸ für eine OH-Gruppe steht und jeweils die übrigen Reste R¹ bis R⁷ die vorstehend genannte Bedeutung haben, können nach üblichen, dem Fachmann bekannten Methoden, wie beispielsweise in Barth, Organikum, Deutscher Verlag der Wissenschaften, 19. Auflage 1993, Seiten 422-424 beschrieben, durchgeführt werden. Die entsprechende Beschreibung wird hiermit als Referenz eingeführt und gilt als Teil der Offenbarung.
Bevorzugt erfolgt die Veresterung durch Umsetzung mit Carbonsäurechloriden der allgemeinen Formel R⁹-(C=O)-Cl oder mit Carbonsäureanhydriden der allgemeinen Formel R⁹-(C=O)-O-(C=O)-R⁹, worin R⁹ jeweils die vorstehend genannte Bedeutung hat.

Das erfindungsgemäße Verfahren kann auch teilweise oder vollständig in semi- oder vollautomatisierter Form als Parallelsynthese einer Mehrzahl von erfindungsgemäßen Cyclopenten-Verbindungen der vorstehend angegebenen allgemeinen Formel I durchgeführt werden.

Die Salze der erfindungsgemäßen substituierten Cyclopenten-Verbindungen der vorstehend angegebenen allgemeinen Formel I können nach üblichen, dem Fachmann bekannten Methoden, beispielsweise durch Umsetzung der jeweiligen Verbindung der allgemeinen Formel I bzw. eines entsprechenden Stereoisomeren, in Form der freien Base mit einer oder mehreren anorganischen Säuren und/oder einer oder mehreren organischen Säuren erhalten werden. Vorzugsweise erfolgt die Umsetzung mit einer Säure ausgewählt aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharin, Cyclohexansulfamidsäure, Aspartam, Sebacinsäuremonomethylester, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, α-Liponsäure, Acetylglycin, Acetylsalicylsäure, Hippursäure und Asparaginsäure.

Die substituierten Cyclopenten-Verbindungen der vorstehenden allgemeinen Formel I sowie ggf. jeweils deren entsprechende Stereoisomere und jeweils deren Salze können nach üblichen, dem Fachmann bekannten Verfahren in Form ihrer Solvate, insbesondere Hydrate, erhalten werden.

Sofern die erfindungsgemäßen substituierten Cyclopenten-Verbindungen der vorstehenden allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Es wurde nun überraschenderweise gefunden, daß sich diese erfindungsgemäßen substituierten Cyclopenten-Verbindungen der allgemeinen Formel I, ggf. ihre entsprechenden Stereoisomeren, jeweils entsprechende Salze und jeweils entsprechende Solvate durch eine hohe Affinität zu dem CGRP-Rezeptor, insbesondere zu dem CGRP1-Rezeptor, auszeichnen und als CGRP-Antagonisten, insbesondere CGRP1-Antagonisten, fungieren. Diese Verbindungen eignen sich daher ausgezeichnet zur Regulation des CGRP-Rezeptors, insbesondere des CGRP1-Rezeptors .

Die erfindungsgemäßen substituierten Cyclopenten-Verbindungen der vorstehenden allgemeinen Formel I und ggf. entsprechende Stereoisomere sowie jeweils die entsprechenden Salze und jeweils die entsprechenden Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine substituierte Cyclopenten-Verbindung der vorstehend angegebenen allgemeinen Formel I, ggf. in Form ihres reinen Stereoisomeren, insbesondere Enantiomeren oder Diastereomeren, ihres Racemates oder in Form einer Mischung der Stereoisomeren, insbesondere der Enantiomeren und/oder Diastereomeren, in einem beliebigen Mischungsverhältnis, und/oder ein entsprechendes physiologisch verträgliches Salz und/oder ein entsprechendes Solvat sowie ggf. eines oder mehrere physiologisch verträgliche Hilfsmittel.

In einer weiteren Ausführungsform kann das erfindungsgemäße Arzneimittel neben wenigstens einer erfindungsgemäßen substituierten Cyclopenten-Verbindung und ggf. einem oder mehreren physiologisch verträglichen Hilfsstoffen auch einen oder mehrere weitere pharmakologisch aktive Wirkstoffe enthalten, die vorzugsweise ausgewählt werden können aus der Gruppe bestehend aus nicht-steroidalen entzündungshemmenden Wirkstoffen (NSAID), Triptanen und Ergotalkaloiden.

Geeignete nicht-steroidale entzündungshemmende Wirkstoffe (NSAID), Triptane und Ergotalkaloide sowie Verfahren zu deren Herstellung sind dem Fachmann bekannt.

Als nicht-steroidale entzündungshemmende Wirkstoffe kommen bevorzugt eine oder mehrere Verbindungen ausgewählt aus der Gruppe bestehend aus Acetylsalicylsäure, Naproxen, Diclofenac, Ibuprofen, Ketoprofen, Piroxicam, Diflunisal, Indomethacin, Tolmetin und Celecoxib in Betracht.

Geeignete Triptane können bevorzugt ausgewählt werden aus der Gruppe bestehend aus Sumatriptan, Eletriptan, Rizatriptan, Zolmitriptan und Naratriptan.

Sofern das erfindungsgemäße Arzneimittel ein oder mehrere Ergotalkaloide aufweist, sind diese bevorzugt Ergotamin und/oder Dihydroergotamin.

Bevorzugt eignet sich das erfindungsgemäße Arzneimitttel zur CGRP-Rezeptor-Regulation, insbesondere zur CGRP1-Rezeptor-Regulation, zur Behandlung und/oder Prophylaxe von Störungen und/oder Krankheiten, die durch den CGRP-Rezeptor, vorzugsweise durch den CGRP-1-Rezeptor, vermittelt werden, zur Prophylaxe und/oder Behandlung von Schmerzen, vorzugsweise von akuten Schmerzen, chronischen Schmerzen, chronisch entzündlichen Schmerzen oder viszeralen Schmerzen, Clusterkopfschmerzen, neurovaskulären Störungen, Migräne, vorzugsweise Migräne mit Aura, Migräne ohne Aura, einfacher Migräne, klassischer Migräne oder komplizierter Migräne, Entzündungen, vorzugsweise Lungenentzündungen, Asthma, Arthritis, Hypertonie, Hypotonie, Tachykardie, nicht-insulinabhängigem Diabetes Mellitus, cardiovaskulären Erkrankungen, Hauterkrankungen und/oder Hautschäden, vorzugsweise von thermisch und/oder strahlenbedingten Hautschäden, besonders bevorzugt von durch Sonnenbrand hervorgerufenen Hautschäden, allergischer Rhinitis, Erkrankungen, die mit einer überschießenden Gefäßerweiterung einhergehen, vorzugsweise Schock oder Sepsis, menopausalen Hitzewallungen oder Opioidtoleranz, vorzugsweise Morphintoleranz.

Ein weiterer Gegenstand der vorliegenden Erfindung ist die Verwendung wenigstens einer substituierten Cyclopenten-Verbindung der allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, und/oder eines entsprechenden Salzes und/oder eines entsprechendes Solvates, zur Herstellung eines Arzneimittels zur CGRP-Rezeptor-Regulation, insbesondere zur CGRP1-Rezeptor-Regulation, zur Behandlung und/oder Prophylaxe von Störungen und/oder Krankheiten, die durch den CGRP-Rezeptor, vorzugsweise durch den CGRP-1-Rezeptor, vermittelt werden, zur Prophylaxe und/oder Behandlung von Schmerzen, vorzugsweise akuten Schmerzen, chronischen Schmerzen, chronisch entzündlichen Schmerzen oder viszeralen Schmerzen, Clusterkopfschmerzen, neurovaskulären Störungen, Migräne, vorzugsweise von Migräne mit Aura, Migräne ohne Aura, einfacher Migräne, klassischer Migräne oder komplizierter Migräne, Entzündungen, vorzugsweise Lungenentzündungen, Asthma, Arthritis, Hypertonie, Hypotonie, Tachykardie, nicht-insulinabhängigem Diabetes Mellitus, cardiovaskulären Erkrankungen, Hauterkrankungen und/oder Hautschäden, vorzugsweise von thermisch und/oder strahlenbedingten Hautschäden, besonders bevorzugt von durch Sonnenbrand hervorgerufenen Hautschäden, allergischer Rhinitis, Erkrankungen, die mit einer überschießenden Gefäßerweiterung einhergehen, vorzugsweise Schock oder Sepsis, menopausalen Hitzewallungen oder Opioidtoleranz, vorzugsweise Morphintoleranz.

Besonders bevorzugt ist die Verwendung wenigstens einer substituierten Cyclopenten-Verbindung der vorstehend angegebenen allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, und/oder eines entsprechenden Salzes und/oder eines entsprechendes Solvates, zur Herstellung eines Arzneimittels zur Prophylaxe und/oder Behandlung von Schmerzen, vorzugsweise akuten Schmerzen, chronischen Schmerzen, chronisch entzündlichen Schmerzen oder viszeralen Schmerzen, Clusterkopfschmerzen, neurovaskulären Störungen, von Migräne, vorzugsweise von Migräne mit Aura, Migräne ohne Aura, einfacher Migräne, klassischer Migräne oder komplizierter Migräne, von Entzündungen, vorzugsweise Lungenentzündungen, oder von Asthma, ganz besonders bevorzugt zur Prophylaxe und/oder Behandlung Schmerzen, vorzugsweise akuten Schmerzen, chronischen Schmerzen, chronisch entzündlichen Schmerzen oder viszeralen Schmerzen, Clusterkopfschmerzen, Migräne mit Aura, Migräne ohne Aura, einfacher Migräne, klassischer Migräne oder komplizierter Migräne.

Ebenfalls bevorzugt ist die Verwendung wenigstens einer substituierten Cyclopenten-Verbindung der vorstehend angegebenen allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, und/oder eines entsprechenden Salzes und/oder eines entsprechendes Solvates, in Kombination mit einem oder mehreren weiteren pharmakologisch aktiven Wirkstoffen, vorzugsweise ausgewählt aus der Gruppe bestehend aus nicht-steroidalen entzündungshemmenden Wirkstoffen, Triptanen und Ergotalkaloiden, zur Herstellung eines Arzneimittels zur Prophylaxe und/oder Behandlung von Schmerzen, vorzugsweise akuten Schmerzen, chronischen Schmerzen, chronisch entzündlichen Schmerzen oder viszeralen Schmerzen, Clusterkopfschmerzen, neurovaskulären Störungen, von Migräne, vorzugsweise von Migräne mit Aura, Migräne ohne Aura, einfacher Migräne, klassischer Migräne oder komplizierter Migräne, von Entzündungen, vorzugsweise Lungenentzündungen, oder von Asthma.

Die erfindungsgemäßen substituierten Cyclopenten-Verbindungen der vorstehenden allgemeinen Formeln I sowie ggf. jeweils entsprechende Stereoisomere können - wie vorstehend beschrieben - auch in Form ihrer physiologisch verträglichen Salze erhalten werden, wobei das erfindungsgemäße Arzneimittel eines oder mehrere Salze einer oder mehrerer dieser Verbindungen aufweisen kann.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren der in dem erfindungsgemäßen Arzneimittel zum Einsatz kommenden substituierten Cyclopenten-Verbindungen der vorstehenden allgemeinen Formel I, jeweils ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, und/oder eines entsprechenden Salzes und/oder eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, in welcher Form das Arzneimittel verabreicht werden soll, beispielsweise oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen.

Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Erfindungsgemäße Cyclopenten-Verbindungen in einem Depot in gelöster Form oder in einem Pflaster, gegebenenfalls unter Zusatz von die Hautpenetration fördernden Mitteln, sind geeignete perkutane Applikationszubereitungen.
Insbesondere oral oder perkutan anwendbare Zubereitungsformen können die jeweiligen substituierten Cyclopenten-Verbindungen auch verzögert freisetzen.

Die Herstellung der erfindungsgemäßen Arzneimittel kann mit Hilfe von üblichen, dem Fachmann bekannten Mitteln, Vorrichtungen, Methoden und Verfahren erfolgen, wie sie beispielsweise in "Remington's Pharmaceutical Sciences", Hrsg. A.R. Gennaro, 17. Auflage, Mack Publishing Company, Easton, Pa. (1985), insbesondere in Teil 8, Kapitel 76-93 beschrieben werden. Die entsprechende Beschreibung wird hiermit als Referenz eingeführt und gilt als Teil der Offenbarung.

Die an den Patienten zu verabreichende Menge der erfindungsgemäßen substituierten Cyclopenten-Verbindungen kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.01 bis 50 mg/kg Körpergewicht des Patienten wenigstens einer erfindungsgemäßen substituierten Cyclopenten-Verbindung appliziert.

### Pharmakologische Methoden:

### (A) Untersuchung der Bindungshemmung von [¹²⁵I]-α-CGRP an den humanen CGRP1-Rezeptor

Es sind verschiedene CGRP-Rezeptor-Subtypen bekannt, die jeweils aus einem Komplex von CRLR (Calcitonin Receptor Like Receptor), RAMP (Receptor-Associated Membrane Protein) und RCP (Receptor Component Protein) bestehen.

Die Untersuchung der Hemmung der Bindung von 3-[¹²⁵I]-iodohistidyl¹⁰-α-CGRP wird an einem humanen CGRP1-Rezeptor durchgeführt, der aus den rekombinanten humanen Bestandteilen (Calcitonin Receptor Like Receptor) CRLR und (Receptor-Associated Membrane Protein) RAMP1 zusammengesetzt ist (Artikel-Nr. ES-420-M9, Euroscreen, Belgien).

Als Radioligand wird 3-[¹²⁵I]-Iodohistidyl¹⁰ Calcitionin Gene Related Peptide (Amersham pharmacia biotech, Code IM 184, 2000 Ci/mmol) eingesetzt.

Die Bindungsstudien werden als SPA-(scintillation proximity assay)-Ansätze mit WGA-beads (wheat germ agglutinin / Amersham pharmacia biotech Code: RPNQ 0001) durchgeführt. Als Inkubationspuffer wird 50 mM Tris pH 7,4 mit 120 mM NaCl, 5 mM KCI und 5 mM MgCl₂ verwendet. Pro Well einer Mikrotitierplatte werden 0,25 µg Protein an Membransuspension von CHO-K1-Zellen, 250 µg Beads und 10 µg Albumin (BSA) sowie der vorstehend genannte Radioligand in einer Konzentration von 100 pM sowie die zu untersuchende erfindungsgemäße Verbindung in einer Konzentration von 10 µM zugegeben

Nach einer Inkubationszeit von 90 Minuten erfolgt die Radioaktivitätsmessung in einem Trilux-Detektor (Wallac, Finnland).

### (B) Untersuchung der Wirkung der erfindungsgemäßen Verbindungen auf die Bildung von cAMP in der humanen Neuroblastom-Zelllinie SK-N-MC.

Zellen der humanen Neuroblastom-Zelllinie SK-N-MC exprimieren endogen CGRP-Rezeptoren hoher Affinität, die eine Aktivierung der Adenylatzyklase vermitten, wie in Van Valen et al., "Calcitonin gene-related peptide (CGRP) receptors are linked to cyclic adenosine monophosphate production in SK-N-MC human neuroblastoma cells", Neuroscience Lett., 119, Seiten 195-198 (1990) beschrieben. Die entsprechende Beschreibung wird hiermit als Referenz eingeführt und gilt als Teil der Offenbarung. Die Messung von 3', 5'-zyklischem AMP (cAMP) erfolgt mit einem kompetitiven Immunoassay wie beschrieben.

### Material und Methoden:

### Zellkultur:

Humane SK-N-MC-Zellen (DSMZ, Braunschweig, Deutschland) werden in Dulbecco's minimal essential medium (DMEM high glucose 4,5 mg/l), welches mit 10 % (Volumen/Volumen) hitzeinaktiviertem fötalen Kälberserum, 2 mM L-Glutamin und 0,1 mM nicht essenziellen Aminosäuren (NEAA, Gibco BRL, Kat.-Nr. 11140-035, L-Alanin 890 mg/l, L-Asparagin 1320 mg/l, L-Asparaginsäure 1330 mg/l, L-Glutaminsäure 1470 mg/l, Glycin 750 mg/l, L-Prolin 1150 mg/l, L-Serin 1050 mg/l) versetzt ist, kultiviert. Das Wachstum erfolgt bei 37 °C, 95 % Luftfeuchtigkeit und 5 % CO₂. Für die cAMP-Bestimmungen werden die Zellen in eienr Zahl von 5 x 10⁴ pro well einer 96-well-Mikrotiterplatte ausgesät und mit den zu testenden Substanzen bei 80 % Konfluenz circa 48 Stunden später inkubiert.

### Messung von zyklischem AMP

Die Messung von 3', 5'-zyklischem AMP (cAMP) erfolgt mit Hilfe eines kompetitiven Immunoassay, wie in Chiulli, A. C. et al., "A novel high throughput chemiluminiscent assay for measurement of cellular cyclic adenosine monophosphate levels, J. Biomol. Screening 5, 239-247, 2000 beschrieben. Die entsprechende Beschreibung wird hiermit als Referenz eigeführt und gilt als Teil der vorliegenden Offenbarung.

Die Messung von 3', 5' zyklischem AMP (cAMP) wird mit dem Tropix^{®} cAMP Screen^{™} chemiluminescent ELISA (Applied Biosystems) durchgeführt. Dieser Immunoassay beinhaltet ein mit Alkalischer Phosphatase (AP)-markiertes cAMP-Konjugat, einen hochspezifischen anti-cAMP Antikörper, vorbeschichtete Mikrotiterplatten, einen cAMP-Standard und das Tropix CSPD^{®}-Substrat mit Lumineszenzverstärker. Das Medium von 80 % konfluenten Zellen in 96-well Platten wird ersetzt durch 110 µl Wachstumsmedium mit 50 µl 4mM Isobutyl-1-methylxanthine-Lösung (IBMX, Sigma, 1 mM Endkonzentration), 20 µl einer Mediumlösung mit der zu untersuchenden Verbindung in 10-fach konzentrierter Form oder Lösungsmittel (Dimethylsulfoxid), und 20 µl von 10-fach konzentriertem CGRP (Calbiochem) oder Lösungsmittel (Essigsäure). Der Anteil an Lösungsmittel soll dabei 0,4 % (Volumen/Volumen) nicht überschreiten. Nach Inkubation für 30 Minuten bei 37°C werden die Zellen pelletiert und das Kulturmedium abgenommen. Das Zellpellet wird in 100 µl Lysepuffer (Applied Biosystems) bei 37 °C bis zur vollständigen Lyse aller Zellen (üblicherweise ca. 30 Minuten, Überprüfung durch mikroskopische Kontrolle) inkubiert. Unmittelbar vor dem Gebrauch wird das cAMP-AP-Konjugat 1:100 Volumen/Volumen dem mit Conjugate Dilution Puffer verdünnt. Zu den Cavitäten einer Assayplatte (Applied Biosystems) werden 60 µl/well der Lysatprobe oder der Standard und 30 µl/well des verdünnten cAMP-AP-Konjugats hinzugegeben, durch wiederholtes Pipettieren gemischt, gefolgt von der Zugabe des anti-cAMP Antikörpers (60 µl/well) und erneutes Homogenisieren durch wiederholtes Pipetieren. Nach 1 Stunde Inkubation bei Raumtemperatur (ca. 25°C) unter Schütteln im Dunkeln wird der Überstand entfernt und die Zellen 6-mal mit dem Waschpuffer (Applied Biosystems) gewaschen.

Zur Detektion werden die Zellen in 100 µl/well der CSPD^{®}/Sapphire-II^{™} RTU Substrate/Enhancer-Lösung für 30 Minuten inkubiert und die Signale in einem Luminometer (MikroBeta Trilux 1450-021, Wallac, Finnland, Serien-Nr. 4500593) bei 1 Sekunde/well gemessen.

Die erfindungsgemäßen substituierten Cyclopenten-Verbindungen werden in 10 µM-Konzentrationen zusammen mit 1 nM CGRP eingesetzt. Dosis-Wirkungs-Kurven werden mit humanem CGRP oder dem antagonistisch wirkenden truncierten Peptid CGRP₍₈₋₃₇₎ (Calbiochem; zusammen mit 1 nM CGRP) bestimmt und das Programm Graph Pad Prism 3.0 für die nicht-lineare Regressionsanalyse verwendet.

### (C) GTPγS-Assay:

Mit Hilfe dieses Assays wird die antagonistische Wirkung der erfindungsgemäßen substituierten Cyclopenten-Verbindungen bestimmt.

Hierzu werden zunächst Membranen von CHO-K1 Zellen (Euroscreen, Brüssel, Belgien), die mit dem humanen calcitonin-receptor related receptor (CRLR, Genbank: U17473) und humanem receptor-associated modifying protein type 1 (RAMP1, Genbank AJ001014) transfiziert sind, zügig aufgetaut, in 20 mM HEPES, 10 mM NaCl, 10 mM MgCl₂, 1 mM EDTA pH 7,4 verdünnt und durch Homogenisieren resuspendiert. Für jeden Testansatz werden 2 µg Membranprotein in 20 mM HEPES pH 7,4, 10 mM MgCl₂, 100 mM NaCl, 1 mM EDTA, 0,1 µM CGRP, 1 µM GDP und der zu prüfenden Substanz in einem Volumen von 150 µl inkubiert. Nach 30-minütiger Inkubationszeit bei Raumtemperatur werden 50 µl 2 nM [35S]GTPγS (Amersham) hinzugefügt und für weitere 30 Minuten bei Raumtemperatur inkubiert und die Assay-Platte für 10 Minuten bei 3200 Umdrehungen pro Minute zentrifugiert. Die gebundene Aktivität wurde mit einem Wallac1450 Microbeta Scintillation Counter (Wallac, Torku, Finnland) bestimmt.

### (D) Bestimmung der CGRP-Rezeptor-Affinität am isolierten, spontan schlagenden Meerschweinchen-Atrium

Es existieren zwei CGRP-Rezeptor-Subtypen, CGRP1 und CGRP2. Funktionell lassen sich diese beiden Subtypen dadurch unterscheiden, daß das Fragment hCGRP₍₈₋₃₇₎ die CGRP1-vermittelte positiv inotrope und chronotrope Wirkung inMeerschweinchen-Atrien stärker hemmt als die CGRP2-vermittelte Hemmung der twitch response am Vas deferens der Ratte. Der selektive CGRP2-Agonist [Cys(ET)^{2,7} ]hCGRPα ist dagegen deutlich stärker wirksam am Ratten-Vas deferens (CGRP2) als am Meerschweinschen-Atrium.(CGRP1), wie in der Literaturveröffentlichung von Y. Dumont et al., "A potent and selective CGRP2 agonist [Cys(ET)2,7 ]hCGRP(8-37): comparison in prototypical CGRP1 and CGRP2 in vitro bioassayss", Can. J. Physiol. Pharmacol. 75: 671-6, 1997 beschrieben. Die entsprechende Literaturbeschreibung wird hiermit als Referenz eingeführt und gilt als Teil der Offenbarung.

Untersuchungen zur Wirkung von CGRP am Meerschweinchen-Atrium sind daher geeignet, in einem funktionellen in-vitro System CGRP1-antagonistische Wirkungen zu quantifizieren.

Zur Bestimmung der CGRP1-antagonistischen Wirkung der erfindungsgemäßen Verbindungen in einem funktionellen in-vitro-System wird an spontan schlagenden Meerschweinchen-Vorhöfen die Zunahme der Herzfrequenz durch humanes α-CGRP (h-α-CGRP) als Parameter für eine CGRP1-agonistische Wirkung ermittelt. Die CGRP1-antagonistische Wirkung von humanem α-CGRP₍₈₋₃₇₎ (h-α-CGRP₍₈₋₃₇₎) an diesem Modell dient als Validierung für die Untersuchung der erfindungsgemäßen Verbindungen.

Die Konzentrationswirkungskurve wird mit h-α-CGRP (Agonist) bestimmt und die zu untersuchenden Verbindungen (Antagonisten) werden jeweils 5 Minuten vorher in das Organbad hinzu gegeben. Hierbei wird h-α-CGRP - gelöst in Dimethylsulfoxid (DMSO) - in Konzentrationen von 1, 2,15, 4,64, 10, 21,5 und 46,4 nM kumulativ in das Organbad gegeben. Die Einwirkungszeit je Konzentrationsstufe beträgt 2 Minuten. Die zu untersuchenden erfindungsgemäßen Verbindungen in verschiedenen Konzentrationen oder Dimethylsulfoxid werden 5 Minuten vor dem Beginn der Konzentrationswirkungskurve mit h-α-CGRP ins Organbad gegeben.

Im folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1:

2-(3,5-Difluor-phenyl)-5-dimethylaminomethyl-3-(4-methansulfonyl-phenyl)-cyclopent-2-enon; Hydrochlorid

Zunächst wurde die Verbindung 2-(3,5-Difluor-phenyl)-3-(4-methansulfonyl-phenyl)-cyclopent-2-enon gemäß der Beschreibung von D. Zhao et al., Tetrahedron 1999, 55, Seite 6001-6018 hergestellt.

Eine Lösung von 1,37 g 2-(3,5-Difluor-phenyl)-3-(4-methansulfonyl-phenyl)-cyclopent-2-enon in 14 ml getrocknetem Acetonitril enthaltend 0,03 ml Acetylchlorid wurde dann mit 0,37 g N,N-Dimethylmethylenimmoniumchlorid versetzt und die so erhaltene Reaktionsmischung für 24 Stunden bei einer Temperatur von 20 °C gerührt. Anschließend wurden 50 ml Diethylether zugegeben und für weitere 3 Stunden bei 20 °C gerührt. Hierbei wurde ein Feststoff erhalten, der durch Absaugen isoliert, mit Diethylether gewaschen und unter vermindertem Druck getrocknet wurde. Es wurden 1,71 g (entsprechend 98,4 % der Theorie) von 2-(3,5-Difluor-phenyl)-5-dimethylaminomethyl-3-(4-methansuffonyl-phenyl)-cyclopent-2-enon; Hydrochlorid in Form nahezu farbloser Kristalle mit einem Schmelzpunkt von 104-106 °C erhalten.

### Beispiele 2-20:

Die erfindungsgemäßen Verbindungen gemäß den Beispielen 2-20 wurden unter Anwendung der gemäß Beispiel 1 ausführlich beschriebenen Verfahrensweise und unter Einsatz der in der nachstehenden Tabelle I angegebenen Edukte der allgemeinen Formel II, die gemäß der Beschreibung von D. Zhao et al., Tetrahedron 1999, 55, Seite 6001-6018 oder von W. Cameron Black et al., J. Med. Chem. 1999, 42 1274-1281 erhalten wurden, hergestellt.

**Tabelle I:**

| **Beispiel** | **eingesetzte Verbindung der allgemeinen Formel II** |
|---|---|
| 2 | 3-(4-Methansulfonyl-phenyl)-2-m-tolyl-cyclopent-2-enon |
| 3 | 2-(3-Hydroxy-phenyl)-3-(4-methansulfonyl-phenyl)-cyclopent-2-enon |
| 4 | 2-(4-Fluor-phenyl)-3-(4-methansulfonyl-phenyl)-cyclopent-2-enon |
| 5 | 2-(3-Fluor-phenyl)-3-(4-methansulfonyl-phenyl)-cyclopent-2-enon |
| 6 | 2-(3-Chlor-phenyl)-3-(4-methansulfonyl-phenyl)-cyclopent-2-enon |
| 7 | 3-(4-Methansulfonyl-phenyl)-2p-tolyl-cyclopent-2-enon |
| 8 | 2-(4-Hydroxy-phenyl)-3-(4-methansulfonyl-phenyl)-cyclopent-2-enon |
| 9 | 3-(4-Methansulfonyl-phenyl)-2-(3-trifluormethyl-phenyl)-cyclopent-2-enon |
| 10 | 3-(4-Methansulfonyl-phenyl)-2-(4-methoxy-phenyl)-cyclopent-2-enon |
| 11 | 2-Benzo[1,3]dioxol-5-yl-3-(4-methansulfonyl-phenyl)-cyclopen-2-enon |
| 12 | 2-(3,5-Dichlor phenyl)-3-(4-methansulfonyl-phenyl)-cyclopent-2-enon |
| 13 | 3-(4-Methansulfonyl-phenyl)-2-(3-methoxy-phenyl)-cyclopent-2-enon |
| 14 | 2-(4-Fluor-3-methyl-phenyl)-3-(4-methansulfonyl-phenyl)- cyclopent-2-enon |
| 15 | 2-(4-tert-Butyl-phenyl)-3-(4-methansulfonyl-phenyl)-cyclopent-2-enon |
| 16 | 2-(3-Ethoxy-phenyl)-3-(4-methansulfonyl-phenyl)-cyclopent-2-enon |
| 17 | 2,3-Bis-(4-methansulfonyl-phenyl)-cyclopent-2-enon |
| 18 | 3-(4-Methansulfonyl-phenyl)-2-naphth-2-yl-cyclopent-2-enon |
| 19 | 2-(3,4-Dimethyl-phenyl)-3-(4-methansulfonyl-phenyl)-cyclopent-2-enon |
| 20 | 2-(3-Isopropyl-phenyl)-3-(4-methansulfonyl-phenyl)-cyclopent-2-enon |

### Beispiel 2:

5-Dimethylaminomethyl-3-(4-methansulfonyl-phenyl)-2-m-tolyl-cyclopent-2-enon, Hydrochlorid. Schmelzpunkt: 148-150°C.

### Beispiel 3:

5-Dimethylaminomethyl-2-(3-hydroxy-phenyl)-3-(4-methansulfonyl-phenyl)-cyclopent-2-enon, Hydrochlorid. Schmelzpunkt: 162-164°C.

### Beispiel 4:

5-Dimethylaminomethyl-2-(4-fluor-phenyl)-3-(4-methansulfonyl-phenyl)-cyclopent-2-enon, Hydrochlorid. Schmelzpunkt: ab 112°C unter Zersetzung.

### Beispiel 5:

5-Dimethylaminomethyl-2-(3-fluor-phenyl)-3-(4-methansulfonyl-phenyl)-cyclopent-2-enon, Hydrochlorid. Schmelzpunkt: 154-158°C.

### Beispiel 6:

2-(3-Chlor-phenyl)-5-dimethylaminomethyl-3-(4-methansulfonyl-phenyl)-cyclopent-2-enon, Hydrochlorid. Schmelzpunkt: 156-158°C.

### Beispiel 7:

5-Dimethylaminomethyl-3-(4-methansulfonyl-phenyl)-2-p-tolyl-cyclopent-2-enon, Hydrochlorid. Schmelzpunkt: 127-129°C.

### Beispiel 8:

5-Dimethylaminomethyl-2-(4-hydroxy-phenyl)-3-(4-methansulfonyl-phenyl)-cyclopent-2-enon, Hydrochlorid. Schmelzpunkt: 98-100°C.

### Beispiel 9:

5-Dimethylaminomethyl-3-(4-methansulfonyl-phenyl)-2-(3-trifluormethylphenyl)-cyclopent-2-enon, Hydrochlorid. Schmelzpunkt: 152-154°C.

### Beispiel 10:

5-Dimethylaminomethyl-3-(4-methansulfonyl-phenyl)-2-(4-methoxyphenyl)-cyclopent-2-enon, Hydrochlorid. Schmelzpunkt ab 92 °C unter Zersetzung.

### Beispiel 11:

2-Benzo[1,3]dioxol-5-yl-5-dimethylaminomethyl-3-(4-methansulfonyl-phenyl)-cyclopent-2-enon, Hydrochlorid. Schmelzpunkt: 149-152°C.

### Beispiel 12:

2-(3,5-Dichlor-phenyl)-5-dimethylaminomethyl-3-(4-methansulfonylphenyl)-cyclopent-2-enon, Hydrochlorid. Schmelzpunkt: 160-163°C.

### Beispiel 13:

5-Dimethylaminomethyl-3-(4-methansulfonyl-phenyl)-2-(3-methoxy-phenyl)-cyclopent-2-enon, Hydrochlorid. Schmelzpunkt: 154-157°C.

### Beispiel 14:

5-Dimethylaminomethyl-2-(4-fluor-3-methyl-phenyl)-3-(4-methansulfonyl-phenyl)-cyclopent-2-enon, Hydrochlorid. Schmelzpunkt: 95-98°C unter Zersetzung.

### Beispiel 15:

2-(4-tert-Butyl-phenyl)-5-dimethylaminomethyl-3-(4-methansulfonyl-phenyl)-cyclopent-2-enon, Hydrochlorid. Schmelzpunkt: 182-184°C.

### Beispiel 16:

5-Dimethylaminomethyl-2-(3-ethoxy-phenyl)-3-(4-methansulfonyl-phenyl)-cyclopent-2-enon, Hydrochlorid. Schmelzpunkt: 149-151°C.

### Beispiel 17:

5-Dimethylaminomethyl-2,3-bis-(4-methansulfonyl-phenyl)-cyclopent-2-enon, Hydrochlorid. Schmelzpunkt: 157-159°C.

### Beispiel 18:

5-Dimethylaminomethyl-3-(4-methansulfonyl-phenyl)-2-naphth-2-yl-cyclopent-2-enon, Hydrochlorid. Schmelzpunkt: 163-165°C.

### Beispiel 19:

5-Dimethylaminomethyl-2-(3,4-dimethyl-phenyl)-3-(4-methansulfonyl-phenyl)-cyclopent-2-enon, Hydrochlorid. Schmelzpunkt: 139-142°C.

### Beispiel 20:

5-Dimethylaminomethyl-2-(3-isopropyl-phenyl)-3-(4-methansulfonyl-phenyl)-cyclopent-2-enon Hydrochlorid. Schmelzpunkt: 131-133°C.

### Beispiel 21:

### 2-(4-tert-Butyl-phenyl)-5-dimethylaminomethyl-3-(4-methansulfonyl-phenyl)-cyclopent-2-enol, Hydrochlorid

Eine Lösung von 1,62g des gemäß Beispiel 15 erhaltenen 2-(4-tert-Butyl-phenyl)-5-dimethylaminomethyl-3-(4-methansulfonyl-phenyl)-cyclopent-2-enon; Hydrochlorids in 15 ml Methanol wurde unter Rühren bei einer Temperatur von 20 ° C portionsweise mit 0,40 g Natriumborhydrid versetzt. Nach vollständiger Zugabe wurde das so erhaltene Reaktionsgemisch noch drei Stunden gerührt und unter vermindertem Druck vollständig eingeengt. Der Rückstand wurde in 50 ml destilliertem Wasser aufgenommen und unter Zugabe von Kaliumcarbonat auf einen pH-Wert von 11 eingestellt. Anschließend wurde dreimal mit jeweils 20 ml Essigsäureethylester extrahiert und die vereinigten Extrakte mit gesättigter Natriumchloridlösung gewaschen, über Natriumsulfat getrocknet und unter vermindertem Druck eingeengt. Der so erhaltene Rückstand wurde säulenchromatographisch an Kieselgel 60 (0,040 - 0,063 mm; E. Merck, Darmstadt, Deutschland) mit einem Gemisch aus Methanol und Essigsäureethylester im Verhältnis von 3:1 als Elutionsmittel gereinigt. Es wurden 0,75 g 2-(4-tert-Butyl-phenyl)-5-dimethylaminomethyl-3-(4-methansulfonyl-phenyl)-cyclopent-2-enol in Form eines farblosen Öls erhalten. Dieses Öl wurde in 25 ml Essigsäurethylester aufgenommen und unter Rühren tropfenweise mit 2 ml einer Lösung von Chlorwasserstoff in Diethylether (5 Gew.-% HCl) versetzt. Es wurde ein kristalliner Feststoff erhalten, der durch Absaugen isoliert, mit Diethylether gewaschen und unter vermindertem Druck getrocknet wurde. Es wurden 0,73 g 2-(4-tert-Butyl-phenyl)-5-dimethylaminomethyl-3-(4-methansulfonyl-phenyl)-cyclopent-2-enol, Hydrochlorid (entsprechend 44,8 % der Theorie) in Form von farblosen Kristallen mit einem Schmelzpunkt von 223 bis 224 °C erhalten.

### Beispiel 22:

### Essigsäure-2-(4-tert-Butyl-phenyl)-5-dimethylaminomethyl-3-(4-methansulfonyl-phenyl)-cyclopent-2-enylester, Hydrochlorid

Es wurden 0,37 g der gemäß Beispiel 21 erhaltenen 2-(4-tert-Butyl-phenyl)-5-dimethylaminomethyl-3-(4-methansulfonyl-phenyl)-cyclopent-2-enol-Verbindung in Form der freien Base in 9 ml getrocknetem Tetrahydrofuran gelöst und die Lösung mit 0,24 ml Pyridin versetzt, Dazu wurden unter Rühren bei 20 °C innerhalb von 30 Minuten zweimal je 0,25 ml Essigsäureanhydrid getropft und die so erhaltene Mischung eine Stunde bei 40 °C gerührt. Anschließend wurde das Reaktionsgemisch auf ein Volumen von ca. 2 ml eingeengt, mit 20 ml destiliertem Wasser versetzt und mit Kaliumcarbonat auf einen pH-Wert von 11 eingestellt. Dann wurde dreimal mit je 10 ml Essigsäureethylester extrahiert, die vereinigten Extrakte mit einer gesättigten Natriumchloridlösung gewaschen, über Natriumsulfat getrocknet und unter vermindertem Druck eingeengt. Der so erhaltene Rückstand wurde säulenchromatographisch an Kieselgel 60 (0,040 - 0,063 mm; E. Merck, Darmstadt, Deutschland) mit einem Gemisch aus Essigsäureethylester und Methanol im Verhältnis von 4:1 als Elutionsmittel gereinigt. Nach Überführung in das entsprechende Hydrochlorid-Salz gemäß der in Beispiel 21 angebenen Methode wurden 0,27 g (entsprechend 61,6 % der theoretisch ermittelten Ausbeute) Essigsäure, 2-(4-tert-Butyl-phenyl)-5-dimethylaminomethyl-3-(4-methansulfonyl-phenyl)-cyclopent-2-enylester, Hydrochlorid in Form von farblosen Kristallen erhalten, die ab 128 °C unter Zersetzung schmolzen.

### Beispiel 23:

### 5-Dimethylaminomethyl-3-(4-methansulfonyl-phenyl)-2m-tolyl-cyclopent-2-enol, Hydrochlorid.

2,28 g 5-Dimethylaminomethyl-3-(4-methansulfonyl-phenyl)-2m-tolyl-cyclopent-2-enon wurden analog zu der in Beispiel 21 beschriebenen Verfahrensweise mit 0,62 g Natriumborhydrid zu 5-Dimethylaminomethyl-3-(4-methansulfonyl-phenyl)-2m-tolyl-cyclopent-2-enol umgesetzt. Nach säulenchromatographischer Reiniung und Fällung des Hydrochlorids mit einer Chlorwasserstoff/Diethylether Mischung wurden 0,95 g (entsprechend 41,4 % der Theorie) in Form von farblosen Kristallen mit einem Schmelzpunkt von 165-167 °C erhalten.

### Pharmakologische Daten:

### (A) Untersuchung der Bindungshemmung von 3-[¹²⁵I]-iodohistidyl¹⁰ α-CGRP an den humanen CGRP1-Rezeptor

Die Hemmung der Bindung von 3-[¹²⁵I]-iodohistidyl¹⁰ Calcitionin Gene Related Peptide an den rekombinanten humanen CGRP1-Rezeptor wurde gemäß der vorstehend beschriebenen Methode ermittelt.

Die erfindungsgemäßen Verbindungen zeigten eine gute bis sehr gute Hemmung der Bindung von 3-[¹²⁵I]-iodohistidyl¹⁰ Calcitionin Gene Related Peptide an den rekombinanten humanen CGRP1-Rezeptor . Die Werte einiger erfindungsgemäßer Verbindungen sind in der nachfolgenden Tabelle (A) wiedergegeben.

**Tabelle A:**

| **Verbindung gemäß Beispiel** | **Hemmung in % [bei einer Konzentration von 10 µM der jeweiligen Verbindung]** |
|---|---|
| 13 | 60 |
| 3 | 66 |
| 5 | 58 |
| 1 | 40 |
| 21 | 26 |

### (B) Untersuchung der Wirkung der erfindungsgemäßen Verbindungen auf die Bildung von cAMP in der humanen Neuroblastom-Zelllinie SK-N-MC:

Gemäß der vorstehend beschriebenen Methode wurde die CGRP Potency (EC₅₀) mit 0,12 nM sowie der IC₅₀-Wert von CGRP8-37 (zusammen mit 1 nM CGRP) mit 1,57 nM zur Validierung der Methode bestimmt.

In drei unabhängigen Versuchen, von denen jeder aus einer Triplikatmessung bestand, zeigten die erfindungsgemäßen substituierten Cyclopenten-Verbindungen eine sehr gute Inhibierung der CGRP-induzierten cAMP-Konzentrationserhöhung.

Die Werte einiger erfindungsgemäßer Verbindungen sind in der nachfolgenden Tabelle (B) wiedergegeben.

**Tabelle B:**

| **Verbindung gemäß Beispiel:** | **Inhibierung der cAMP-Bildung (bei 1 nM CGRP) [in %]:** |
|---|---|
| 13 | 88 |
| 3 | 86 |
| 5 | 82 |
| 1 | 71 |

### (C) GTPγS-Assay

Die Inhibierung des CGRP-induzierten Einbaus von [³⁵S]GTPγS in Membranen wurde gemäß der vorstehend beschriebenen Methode ermittelt.

Die erfindungsgemäßen Verbindungen vermögen den CGRP-induzierten Einbau von [³⁵S]GTPγS in die Membranen zu inhibieren und wirken damit als CGRP-Rezeptor Antagonisten.

Die Werte einiger erfindungsgemäßer Verbindungen sind in der nachfolgenden Tabelle (C) wiedergegeben:

**Tabelle (C):**

| **Verbindung gemäß Beispiel [Konzentration jeweils 12,5 µM]** | **% Hemmung des CGRP-induzierten [35S]GTPγS-Einbaus [Konzentration-CGRP von 0,1 µM]** |
|---|---|
| 1 | 65,1 |
| 3 | 65,1 |
| 13 | 63,9 |
| 5 | 62,4 |

### (D) Bestimmung der CGRP-Rezeptor-Affinität am isolierten, spontan

### schlagenden Meerschweinchen-Atrium:

Die Validierung sowie die Untersuchung der erfindungsgemäßen substituierten Cyclopenten-Verbindungen auf ihre antagonistische Wirkung erfolgte gemäß der vorstehend beschriebenen Methode.

Im Validierungsexperiment führte h-α-CGRP₍₈₋₃₇₎ in Konzentrationen von 30, 100 und 300 nM zu einer konzentrationsabhängigen Reduktion der Zunahme der Schlagfrequenz der Vorhöfe, die durch h-α-CGRP stimuliert wurden. Die Konzentration von h-α-CGRP₍₈₋₃₇₎, die eine halbmaximale Wirkung erzielte (IC₅₀) betrug 0,019 µM.

In Anwesenheit von Vehikellösung steigerte h-α-CGRP die Schlagfrequenz der isolierten Atrien um ca. 35 %. Diese positive chronotrope Wirkung, die über die CGRP1-Rezeptoren vermittelt wird, wurde durch die erfindungsgemäßen substituierten Cyclopenten-Verbindungen mit CGRP-antagonistischer Wirkung konzentrationsabhängig reduziert.

Die IC₅₀-Werte für die CGRP-antagonistische Wirkung einiger erfindungsgemäßer Verbindungen sind in der folgenden Tabelle (D) wiedergegeben:

**Tabelle D:**

| **Verbindung gemäß Beispiel:** | **IC₅₀-Wert [in µM]** |
|---|---|
| 13 | 32 |
| 3 | 77 |
| 5 | 25 |
| 1 | 13 |

## Patentansprüche

1. Substituierte Cyclopenten-Verbindungen der allgemeinen Formel I, 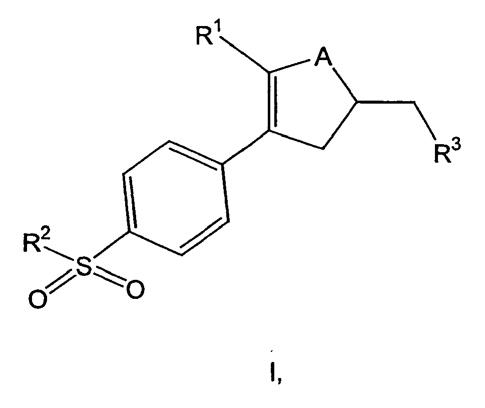 worin
R¹ für einen ggf. wenigstens einfach substituierten Phenyl-Rest steht, der mit einem ggf. wenigstens einfach substituierten, gesättigten, ungesättigten oder aromatischen, ggf. wenigstens ein Heteroatom als Ringglied aufweisenden monozyklischen Ringsystem kondensiert sein kann,
R² für einen geradkettigen oder verzweigten Alkyl-Rest oder für eine -NH₂-Gruppe steht,
R³ für eine -NR⁴R⁵-Gruppe oder eine -NR⁶R⁷-Gruppe steht,
R⁴ und R⁵, gleich oder verschieden, jeweils für Wasserstoff oder einen geradkettigen oder verzweigten Alkyl-Rest stehen, mit der Maßgabe, daß nicht beide Reste R⁴ und R⁵ gleichzeitig Wasserstoff bedeuten,
R⁶ und R⁷ gemeinsam mit dem sie verbrückenden Stickstoffatom als Ringglied einen gesättigten, ggf. wenigstens einfach substituierten, ggf. wenigstens ein weiteres Heteroatom als Ringglied aufweisenden heterozyklischen Rest bilden, A für eine C(=O)-Gruppe oder für eine C(H)(R⁸)-Gruppe steht,
R⁸ für eine OH-Gruppe oder für eine -O-(C=O)-R⁹ -Gruppe steht,
R⁹ für einen geradkettigen oder verzweigten Alkyl-Rest steht,
jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

2. Verbindungen gemäß Anspruch 1, **dadurch gekennzeichnet, daß** R¹ für einen ggf. wenigstens einfach substituierten Phenyl-Rest steht, der mit einem ggf. wenigstens einfach substituierten, gesättigten, ungesättigten oder aromatischen, ggf. wenigstens ein Heteroatom als Ringglied aufweisenden, 5-oder 6-gliedrigen monozyklischen Ringsystem kondensiert sein kann, vorzugsweise für einen ggf. wenigstens einfach substituierten Phenyl-, Naphthyl- oder Benzo[1,3]-dioxol-Rest steht.

3. Verbindungen gemäß Anspruch 1 oder 2, **dadurch gekennzeichnet, daß** R² für einen geradkettigen oder verzweigten C₁₋₆-Alkyl-Rest oder für eine -NH₂-Gruppe, vorzugsweise für einen geradkettigen oder verzweigten C₁₋₄-Alkyl-Rest oder für eine NH₂-Gruppe, besonders bevorzugt für einen Methyl-Rest, einen Ethyl-Rest oder für eine NH₂-Gruppe, ganz besonders bevorzugt für einen Methyl-Rest steht.

4. Verbindungen gemäß einem der Ansprüche 1 bis 3, **dadurch gekennzeichnet, daß** R⁴ und R⁵, gleich oder verschieden, jeweils für Wasserstoff oder einen geradkettigen oder verzweigten C₁₋₆-Alkyl-Rest, vorzugsweise jeweils für Wasserstoff oder einen geradkettigen oder verzweigten C₁₋₄-Alkyl-Rest, besonders bevorzugt jeweils für Wasserstoff, einen Methyl-Rest oder einen Ethyl-Rest, ganz besonders bevorzugt beide gleichzeitig für einen Methyl-Rest stehen.

5. Verbindungen gemäß einem der Ansprüche 1 bis 4, **dadurch gekennzeichnet, daß** R⁶ und R⁷ gemeinsam mit dem sie verbrückenden Stickstoffatom als Ringglied einen gesättigten, ggf. wenigstens einfach substituierten, ggf. wenigstens ein weiteres Heteroatom als Ringglied aufweisenden, 5- bis 8-gliedrigen heterozyklischen Rest, vorzugsweise einen ggf. wenigstens einfach substituierten Pyrrolidin-, Piperidin-, Hexamethylenimin- oder Morpholin-Ring bilden.

6. Verbindungen gemäß einem der Ansprüche 1 bis 5, **dadurch gekennzeichnet, daß** A für eine -C(=O)-Gruppe steht.

7. Verbindungen gemäß einem der Ansprüche 1 bis 6, **dadurch gekennzeichnet, daß** R⁹ für einen geradkettigen oder verzweigten C₁₋₆-Alky/-Rest, vorzugsweise für einen geradkettigen oder verzweigten C₁₋₄-Alkyl-Rest , besonders bevorzugt für einen Methyl-Rest steht.

8. Verbindungen gemäß einem oder mehreren der Ansprüche 1 bis 7, **dadurch gekennzeichnet, daß**
R¹ für einen Phenyl-, Naphtyl- oder Benzo[1,3]dioxolyl-Rest steht, der jeweils unsubstituiert oder mit 1, 2, 3, 4 oder 5 Substituenten unabhängig voneinander ausgewählt aus der Gruppe bestehend aus -F; -Cl; -Br; -I; -CF₃; -OH; Methyl; Ethyl; n-Propyl; iso-Propyl; n-Butyl; sec-Butyl; iso-Butyl; tert-Butyl; Methoxy; Ethoxy; n-Propyloxy; iso-Propyloxy; n-Butyloxy; sec-Butyloxy; iso-Butyloxy; tert-Butyloxy; -SO₂-methyl -SO₂-ethyl; -SO₂-n-propyl -SO₂-iso-propyl -SO₂-n-butyl; -SO₂-sec-butyl -SO₂-iso-butyl; -SO₂-tert-butyl und -SO₂-NH₂ substituiert ist,
R² für eine Methyl-Gruppe oder für eine -NH₂-Gruppe steht,
R³ für eine -NR⁴R⁵-Gruppe steht,
R⁴ und R⁵ jeweils für eine Methyl-Gruppe stehen;
A für eine C(=O)-Gruppe oder für eine C(H)(R⁸)-Gruppe steht,
R⁸ für eine OH-Gruppe oder für eine -O-(C=O)-R⁹ -Gruppe steht,
R⁹ für eine Methyl- oder Ethyl-Gruppe steht;
jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

9. Verbindungen gemäß Anspruch 8, **dadurch gekennzeichnet, daß** R¹ für einen Rest steht, der ausgewählt ist aus der Gruppe bestehend aus
2-Fluor-phenyl; 3-Fluor-phenyl; 4-Fluor-phenyl; 2-Chlor-phenyl; 3-Chlor-phenyl; 4-Chlor-phenyl; 2-Brom-phenyl; 3-Brom-phenyl; 4-Brom-phenyl; 3,5-Difluorphenyl; 3,5-Dichlor-phenyl; 3,5-Dibrom-phenyl; 2-Trifluormethyl-phenyl; 3-Trifluormethyl-phenyl; 4-Trifluormethyl-phenyl; 4-Fluor-3-Methyl-phenyl; o-Tolyl; m-Tolyl; p-Tolyl; 2-Ethyl-phenyl; 3-Ethyl-phenyl; 4-Ethyl-phenyl; 2-iso-Propyl-phenyl; 3-iso-Propyl-phenyl; 4-iso-Propyl-phenyl; 2-tert-Butyl-phenyl; 3-tert-Butyl-phenyl; 4-tert-Butyl-phenyl; 3,4-Dimethyl-phenyl; 2-Hydroxy-phenyl; 3-Hydroxy-phenyl; 4-Hydroxy-phenyl; 2-Methoxy-phenyl; 3-Methoxy-phenyl; 4-Methoxy-phenyl; 2-Ethoxy-phenyl; 3-Ethoxy-phenyl; 4-Ethoxy-phenyl; 2-Methansulfonyl-phenyl; 3-Methansulfonyl-phenyl; 4-Methansulfonyl-phenyl; Benzo[1,3}dioxo-5-yl; Naphth-1-yl und Naphth-2-yl.

10. Verbindungen gemäß einem oder mehreren der Ansprüche 1 bis 9 ausgewählt aus der Gruppe bestehend aus
2-(3,5-Difluor-phenyl)-5-dimethylaminomethyl-3-(4-methansulfonyl-phenyl)-cyclopent-2-enon,
5-Dimethylaminomethyl-3-(4-methansulfonyl-phenyl)-2-m-tolyl-cyclopent-2-enon,
5-Dimethylaminomethyl-2-(3-hydroxy-phenyl)-3-(4-methansulfonyl-phenyl)-cyclopent-2-enon,
5-Dimethylaminomethyl-2-(4-fluor-phenyl)-3-(4-methansulfonyl-phenyl)-cyclopent-2-enon,
5-Dimethylaminomethyl-2-(3-fluor-phenyl)-3-(4-methansulfonyl-phenyl)-cyclopent-2-enon,
2-(3-Chlor-phenyl)-5-dimethylaminomethyl-3-(4-methansulfonyl-phenyl)-cyclopent-2-enon,
5-Dimethylaminomethyl-3-(4-methansulfonyl-phenyl)-2-p-tolyl-cyclopent-2-enon,
5-Dimethylaminomethyl-2-(4-hydroxy-phenyl)-3-(4-methansulfonyl-phenyl)-cyclopent-2-enon,
5-Dimethylaminomethyl-3-(4-methansulfonyl-phenyl)-2-(3-trifluormethylphenyl)-cyclopent-2-enon,
5-Dimethylaminomethyl-3-(4-methansulfonyl-phenyl)-2-(4-methoxy-phenyl)-cyclopent-2-enon,
2-Benzo[1,3]dioxol-5-yl-5-dimethylaminomethyl-3-(4-methansulfonyl-phenyl)-cyclopent-2-enon,
2-(3,5-Dichlor-phenyl)-5-dimethylaminomethyl)-3-(4-methansulfonyl-phenyl)-cyclopent-2-enon,
5-Dimethylaminomethyl-3-(4-methansulfonyl-phenyl)-2-(3-methoxy-phenyl)-cyclopent-2-enon,
5-Dimethylaminomethyl-2-(4-fluor-3-methyl-phenyl)-3-(4-methansulfonyl-phenyl)-cyclopent-2-enon,
2-(4-tert-Butyl-phenyl)-5-dimethylaminomethyl-3-(4-methansulfonyl-phenyl)-cyclopent-2-enon,
5-Dimethylaminomethyl-2-(3-ethoxy-phenyl)-3-(4-methansulfonyl-phenyl)-cyclopent-2-enon,
5-Dimethylaminomethyl-2,3-bis-(4-methansulfonyl-phenyl)-cyclopent-2-enon,
5-Dimethylaminomethyl-3-(4-methansulfonyl-phenyl)-2-naphth-2-yl-cyclopent-2-enon,
5-Dimethylaminomethyl-2-(3,4-dimethyl-phenyl)-3-(4-methansulfonyl-phenyl)-cyclopent-2-enon,
5-Dimethylaminomethyl-2-(3-isopropyl-phenyl)-3-(4-methansulfonyl-phenyl)-cyclopent-2-enon,
2-(4-tert-Butyl-phenyl)-5-dimethylaminomethyl-3-(4-methansulfonyl-phenyl)-cyclopent-2-enol,
Essigsäure-2-(4-tert-Butyl-phenyl)-5-dimethylaminomethyl-3-(4-methansulfonyl-phenyl)-cyclopent-2-enylester,
5-Dimethylaminomethyl-3-(4-methansulfonyl-phenyl)-2m-tolyl-cyclopent-2-enol,
und jeweils entsprechenden Salzen, vorzugsweise Hydrochloriden, und jeweils entsprechenden Solvaten, vorzugsweise Hydraten.

11. Verfahren zur Herstellung substituierter Cyclopenten-Verbindungen gemäß den Ansprüchen 1 bis 10, **dadurch gekennzeichnet, daß** wenigstens eine Verbindung der allgemeinen Formel II 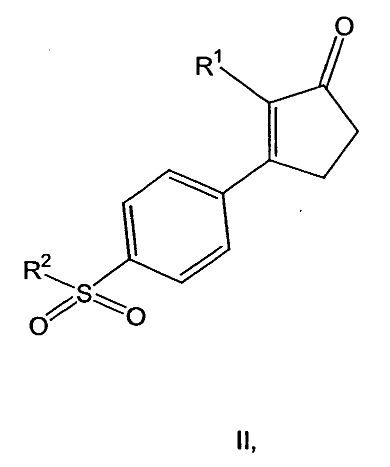 worin R¹ und R² die Bedeutung gemäß den Ansprüchen 1-10 haben, durch Umsetzung mit Formaldehyd und/oder Paraformaldehyd und wenigstens einer substituierten Aminverbindung der allgemeinen Formel IIIa 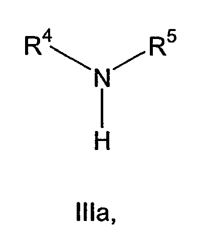 worin R⁴ und R⁵ die Bedeutung gemäß den Ansprüchen 1 bis 10 haben, oder wenigstens einer substituierten Aminverbindung der allgemeinen Formel IIIb, 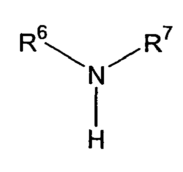 worin R⁶ und R⁷die Bedeutung gemäß den Ansprüchen 1 bis 10 haben, oder durch Umsetzung mit wenigstens einer Methylenimmonium-Verbindung der allgemeinen Formel IVa 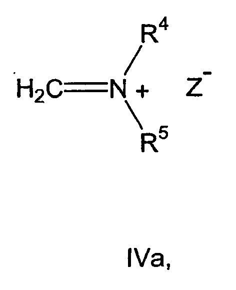 worin R⁴ und R⁵ die Bedeutung gemäß den Ansprüchen 1 bis 10 haben und Z für ein Chlor-, Brom- oder lodatom steht, oder mit wenigstens einer Methylenimmonium-Verbindung der allgemeinen Formel IVb, worin 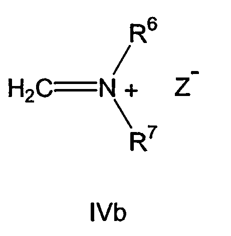 worin R⁶ und R⁷ die Bedeutung gemäß den Ansprüchen 1 bis 10 haben und Z für ein Chlor-, Brom- oder lodatom steht, in eine Verbindung der allgemeinen Formel I gemäß den Ansprüchen 1 bis 10, worin R¹ bis R³ die Bedeutung gemäß den Ansprüchen 1 bis 10 haben und A für eine -(C=O)-Gruppe steht, übergeführt, ggf. gereinigt, ggf. isoliert, und ggf. durch Umsetzung mit wenigstens einem Reduktionsmittel in eine Verbindung der allgemeinen Formel I gemäß den Ansprüchen 1 bis 10, worin R¹ bis R³ die Bedeutung gemäß den Ansprüchen 1 bis 10 haben, und A für eine C(H)(R⁸)-Gruppe und R⁸ für eine Hydroxy-Gruppe steht, übergeführt, ggf. gereinigt, ggf. isoliert,
und diese ggf. durch Umsetzung mit wenigstens einem Veresterungsreagenz in eine Verbindung der allgemeinen Formel I, worin R¹ bis R³ die Bedeutung gemäß den Ansprüchen 1 bis 10 haben, A für eine C(H)(R⁸)-Gruppe und R⁸ für eine - O(C=O)-R⁹-Gruppe steht, worin R⁹ die Bedeutung gemäß den Ansprüchen 1 bis 10 hat, übergeführt, diese ggf. reinigt und ggf. isoliert wird.

12. Arzneimittel enthaltend wenigstens eine Cyclopenten-Verbindung gemäß den Ansprüchen 1 bis 10 sowie ggf. wenigstens einen weiteren pharmakologisch aktiven Wirkstoff und ggf. einen oder mehrere physiologisch verträgliche Hilfsstoffe.

13. Arzneimittel gemäß Anspruch 12, **dadurch gekennzeichnet, daß** der weitere pharmakologisch aktive Wirkstoff ausgewählt ist aus der Gruppe bestehend aus nicht-steroidalen entzündungshemmenden Wirkstoffen (NSAID), Triptanen und Ergotalkaloiden.

14. Arzneimittel gemäß Anspruch 13, **dadurch gekennzeichnet, daß** der nicht-steroidale entzündungshemmende Wirkstoff ausgewählt ist aus der Gruppe bestehend aus Acetylsalicylsäure, Naproxen, Diclofenac, Ibuprofen, Ketoprofen, Piroxicam, Diflunisal, Indomethacin, Tolmetin und Celecoxib.

15. Arzneimittel gemäß Anspruch 13, **dadurch gekennzeichnet, daß** das Triptan ausgewählt ist aus der Gruppe bestehend aus Sumatriptan, Eletriptan, Rizatriptan, Zolmitriptan und Naratriptan.

16. Arzneimittel gemäß Anspruch 13, **dadurch gekennzeichnet, daß** als Ergotalkaloid Ergotamin oder Dihydroergotamin vorliegt.

17. Arzneimittel gemäß einem oder mehreren der Ansprüche 12-16 zur CGRP-Rezeptor-Regulation, vorzugsweise zur CGRP1-Rezeptor-Regulation, zur Behandlung und/oder Prophylaxe von Störungen und/oder Krankheiten, die durch den CGRP-Rezeptor, vorzugsweise durch den CGRP-1-Rezeptor, vermittelt werden, zur Prophylaxe und/oder Behandlung von Schmerzen, vorzugsweise akuten Schmerzen, chronischen Schmerzen, chronisch entzündlichen Schmerzen oder viszeralen Schmerzen, Clusterkopfschmerzen, neurovaskulären Störungen, Migräne, vorzugsweise von Migräne mit Aura, Migräne ohne Aura, einfacher Migräne, klassischer Migräne oder komplizierter Migräne, Entzündungen, vorzugsweise Lungenentzündungen, Asthma, Arthritis, Hypertonie, Hypotonie, Tachykardie, nicht-insulinabhängigem Diabetes Mellitus, cardiovaskulären Erkrankungen, Hauterkrankungen und/oder Hautschäden, vorzugsweise von thermisch und/oder strahlenbedingten Hautschäden, besonders bevorzugt von durch Sonnenbrand hervorgerufenen Hautschäden, allergischer Rhinitis, Erkrankungen, die mit einer überschießenden Gefäßerweiterung einhergehen, vorzugsweise Schock oder Sepsis, menopausalen Hitzewallungen oder Opioidtoleranz, vorzugsweise Morphintoleranz.

18. Verwendung wenigstens einer Verbindung gemäß den Ansprüchen 1 bis 10 zur Herstellung eines Arzneimittels zur CGRP-Rezeptor-Regulation, vorzugsweise zur CGRP-1-Rezeptor-Regulation.

19. Verwendung wenigstens einer Verbindung gemäß den Ansprüchen 1 bis 10 zur Herstellung eines Arzneimittels zur Behandlung und/oder Prophylaxe von Störungen und/oder Krankheiten, die durch den CGRP-Rezeptor, vorzugsweise durch den CGRP-1-Rezeptor, vermittelt werden.

20. Verwendung gemäß Anspruch 19 zur Prophylaxe und/oder Behandlung von Schmerzen, vorzugsweise akuten Schmerzen, chronischen Schmerzen, chronisch entzündlichen Schmerzen oder viszeralen Schmerzen.

21. Verwendung gemäß Anspruch 19 zur Behandlung und/oder Prophylaxe von Clusterkopfschmerzen.

22. Verwendung gemäß Anspruch 19 zur Prophylaxe und/oder Behandlung von neurovaskulären Störungen.

23. Verwendung gemäß Anspruch 19 zur Prophylaxe und/oder Behandlung von Migräne, vorzugsweise von Migräne mit Aura, Migräne ohne Aura, einfacher Migräne, klassischer Migräne oder komplizierter Migräne.

24. Verwendung gemäß Anspruch 19 zur Prophylaxe und/oder Behandlung von Entzündungen, vorzugsweise Lungenentzündungen.

25. Verwendung gemäß Anspruch 19 zur Prophylaxe und/oder Behandlung von Asthma.

26. Verwendung gemäß Anspruch 19 zur Prophylaxe und/oder Behandlung von Arthritis.

27. Verwendung gemäß Anspruch 19 zur Prophylaxe und/oder Behandlung von Hypertonie.

28. Verwendung gemäß Anspruch 19 zur Prophylaxe und/oder Behandlung von Hypotonie.

29. Verwendung gemäß Anspruch 19 zur Prophylaxe und/oder Behandlung von Tachykardie.

30. Verwendung gemäß Anspruch 19 zur Prophylaxe und/oder Behandlung von nicht-insulinabhängigem Diabetes Mellitus.

31. Verwendung gemäß Anspruch 19 zur Prophylaxe und/oder Behandlung von cardiovaskulären Erkrankungen.

32. Verwendung gemäß Anspruch 19 zur Prophylaxe und/oder Behandlung von Hauterkrankungen und/oder Hautschäden, vorzugsweise von thermisch und/oder strahlenbedingten Hautschäden, besonders bevorzugt von durch Sonnenbrand hervorgerufenen Hautschäden.

33. Verwendung gemäß Anspruch 19 zur Prophylaxe und/oder Behandlung von allergischer Rhinitis.

34. Verwendung gemäß Anspruch 19 zur Prophylaxe und/oder Behandlung von Erkrankungen, die mit einer überschießenden Gefäßerweiterung einhergehen, vorzugsweise Schock oder Sepsis.

35. Verwendung gemäß Anspruch 19 zur Prophylaxe und/oder Behandlung von menopausalen Hitzewallungen.

36. Verwendung gemäß Anspruch 19 zur Verringerung von Opioidtoleranz, vorzugsweise Morphintoleranz.

## Claims

1. Substituted cyclopentene compounds of the general formula I, 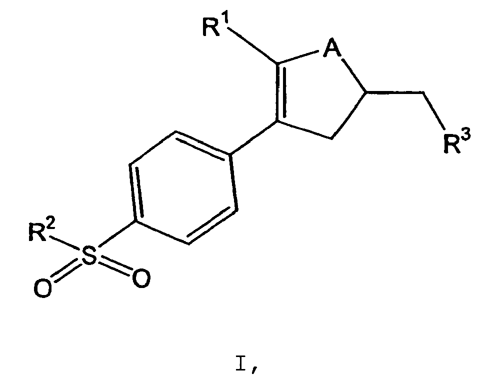 in which
R¹ denotes an optionally at least mono-substituted phenyl residue, which may be fused with an optionally at least mono-substituted, saturated, unsaturated or aromatic monocyclic ring system optionally comprising at least one heteroatom as a ring member,
R² denotes a linear or branched alkyl residue or an - NH₂ group,
R³ denotes an -NR⁴R⁵ group or an -NR⁶R⁷ group,
R⁴ and R⁵, identical or different, in each case denote hydrogen or a linear or branched alkyl residue, with the proviso that the two residues R⁴ and R⁵do not simultaneously mean hydrogen,
R⁶ and R⁷, together with the nitrogen atom bridging them as a ring member, form a saturated, optionally at least mono-substituted, heterocyclic residue optionally comprising at least one further heteroatom as a ring member,
A denotes a C(=O) group or a C (H) (R⁸) group,
R⁸ denotes an OH group or an -O-(C=O)-R⁹ group,
R⁹ denotes a linear or branched alkyl residue,
in each case optionally in the form of the pure stereoisomers thereof, in particular enantiomers or diastereomers, the racemates thereof or in the form of mixtures of the stereoisomers, in particular the enantiomers and/or diastereomers, in any desired mixing ratio, or in each case in the form of corresponding salts or in each case in the form of corresponding solvates.

2. Compounds according to claim 1, **characterised in that** R¹ denotes an optionally at least mono-substituted phenyl residue, which may be fused with an optionally at least mono-substituted, saturated, unsaturated or aromatic 5- or 6-membered monocyclic ring system optionally comprising at least one heteroatom as a ring member and preferably denotes an optionally at least mono-substituted phenyl, naphthyl or benzo[1,3]dioxole residue.

3. Compounds according to claim 1 or 2, **characterised in that** R² denotes a linear or branched C₁₋₆ alkyl residue or an -NH₂ group, preferably a linear or branched C₁₋₄ alkyl residue or an NH₂ group, particularly preferably a methyl residue, an ethyl residue or an NH₂ group, very particularly preferably a methyl residue.

4. Compounds according to any one of claims 1 to 3, **characterised in that** R⁴ and R⁵, identical or different, in each case denote hydrogen or a linear or branched C₁₋₆ alkyl residue, preferably in each case hydrogen or a linear or branched C₁₋₄ alkyl residue, particularly preferably in each case hydrogen, a methyl residue or an ethyl residue, very particularly preferably both simultaneously denote a methyl residue.

5. Compounds according to any one of claims 1 to 4, **characterised in that** R⁶ and R⁷, together with the nitrogen atom bridging them as a ring member, form a saturated, optionally at least mono-substituted, 5- to 8-membered heterocyclic residue optionally comprising at least one further heteroatom as a ring member, preferably an optionally at least mono-substituted pyrrolidine, piperidine, hexamethylenimine or morpholine ring.

6. Compounds according to any one of claims 1 to 5, **characterised in that** A denotes a -C(=O) group.

7. Compounds according to any one of claims 1 to 6, **characterised in that** R⁹ denotes a linear or branched C₁₋₆ alkyl residue, preferably a linear or branched C₁₋₄ alkyl residue, particularly preferably a methyl residue.

8. Compounds according to one or more of claims 1 to 7,
**characterised in that**
R¹ denotes a phenyl, naphthyl or benzo[1,3]dioxolyl residue, which is in each case unsubstituted or substituted with 1, 2, 3, 4 or 5 substituents mutually independently selected from the group consisting of F; Cl; Br; I; -CF₃; -OH; methyl; ethyl; n-propyl; iso-propyl; n-butyl; sec-butyl; iso-butyl; tert.-butyl; methoxy; ethoxy; n-propyloxy; iso-propyloxy; n-butyloxy; sec-butyloxy; iso-butyloxy; tert-butyloxy; -SO₂-methyl -SO₂-ethyl; -SO₂-n-propyl; -SO₂-iso-propyl; -SO₂-n-butyl; -SO₂-sec-butyl; -SO₂-iso-butyl; -SO₂-tert.-butyl and -SO₂-NH₂,
R² denotes a methyl group or an -NH₂ group,
R³ denotes an -NR⁴R⁵ group,
R⁴ and R⁵ in each case denote a methyl group;
A denotes a C (=O) group or a C (H) (R⁸) group, R⁸ denotes an OH group or a -O-(C=O)-R⁹ group, R⁹ denotes a methyl or ethyl group;
in each case optionally in the form of the pure stereoisomers thereof, in particular enantiomers or diastereomers, the racemates thereof or in the form of mixtures of the stereoisomers, in particular the enantiomers and/or diastereomers, in any desired mixing ratio, or in each case in the form of corresponding salts or in each case in the form of corresponding solvates.

9. Compounds according to claim 8, **characterised in that** R¹ denotes a residue which is selected from the group consisting of
2-fluorophenyl; 3-fluorophenyl; 4-fluorophenyl;
2-chlorophenyl; 3-chlorophenyl; 4-chlorophenyl;
2-bromophenyl; 3-bromophenyl; 4-bromophenyl;
3,5-difluorophenyl; 3,5-dichlorophenyl;
3,5-dibromophenyl; 2-trifluoromethylphenyl; 3-trifluoromethylphenyl; 4-trifluoromethylphenyl; 4-fluoro-3-methylphenyl; o-tolyl; m-tolyl; p-tolyl; 2-ethylphenyl; 3-ethylphenyl; 4-ethylphenyl; 2-iso-propylphenyl; 3-iso-propylphenyl; 4-iso-propylphenyl; 2-tert.-butylphenyl; 3-tert.-butylphenyl; 4-tert.-butylphenyl; 3,4-dimethylphenyl; 2-hydroxyphenyl; 3-hydroxyphenyl; 4-hydroxyphenyl; 2-methoxyphenyl; 3-methoxyphenyl; 4-methoxyphenyl; 2-ethoxyphenyl; 3-ethoxyphenyl; 4-ethoxyphenyl; 2-methanesulfonylphenyl; 3-methanesulfonylphenyl; 4-methanesulfonylphenyl; benzo[1,3]dioxo-5-yl; naphth-1-yl and naphth-2-yl.

10. Compounds according to one or more of claims 1 to 9 selected from the group consisting of
2-(3,5-difluorophenyl)-5-dimethylaminomethyl-3-(4-methanesulfonylphenyl)-cyclopent-2-enone,
5-dimethylaminomethyl-3-(4-methanesulfonylphenyl)-2-m-tolylcyclopent-2-enone,
5-dimethylaminomethyl-2-(3-hydroxyphenyl)-3-(4-methanesulfonylphenyl)-cyclopent-2-enone,
5-dimethylaminomethyl-2-(4-fluorophenyl)-3-(4-methanesulfonylphenyl)-cyclopent-2-enone,
5-dimethylaminomethyl-2-(3-fluorophenyl)-3-(4-methanesulfonylphenyl)-cyclopent-2-enone,
2-(3-chlorophenyl)-5-dimethylaminomethyl-3-(4-methanesulfonylphenyl)-cyclopent-2-enone, 5-dimethylaminomethyl-3-(4-methanesulfonylphenyl)-2-p-tolylcyclopent-2-enone,
5-dimethylaminomethyl-2-(4-hydroxyphenyl)-3-(4-methanesulfonylphenyl)-cyclopent-2-enone,
5-dimethylaminomethyl-3-(4-methanesulfonylphenyl)-2-(3-trifluoromethylphenyl)-cyclopent-2-enone,
5-dimethylaminomethyl-3-(4-methanesulfonylphenyl)-2-(4-methoxyphenyl)-cyclopent-2-enone,
2-benzo[1,3]dioxol-5-yl-5-dimethylaminomethyl-3-(4-methanesulfonylphenyl)-cyclopent-2-enone,
2-(3,5-dichlorophenyl)-5-dimethylaminomethyl-3-(4-methanesulfonylphenyl)-cyclopent-2-enone,
5-dimethylaminomethyl-3-(4-methanesulfonylphenyl)-2-(3-methoxyphenyl)-cyclopent-2-enone,
5-dimethylaminomethyl-2-(4-fluoro-3-methylphenyl)-3-(4-methanesulfonylphenyl)-cyclopent-2-enone,
2-(4-tert-butylphenyl)-5-dimethylaminomethyl-3-(4-methanesulfonylphenyl)-cyclopent-2-enone,
5-dimethylaminomethyl-2-(3-ethoxyphenyl)-3-(4-methanesulfonylphenyl)-cyclopent-2-enone,
5-dimethylaminomethyl-2,3-bis-(4-methanesulfonylphenyl)-cyclopent-2-enone,
5-dimethylaminomethyl-3-(4-methanesulfonylphenyl)-2-naphth-2-ylcyclopent-2-enone,
5-dimethylaminomethyl-2-(3,4-dimethylphenyl)-3-(4-methanesulfonylphenyl)-cyclopent-2-enone, 5-dimethylaminomethyl-2-(3-isopropylphenyl)-3-(4-methanesulfonylphenyl)-cyclopent-2-enone,
2-(4-tert-butylphenyl)-5-dimethylaminomethyl-3-(4-methanesulfonylphenyl)-cyclopent-2-enol,
acetic acid 2-(4-tert-butylphenyl)-5-dimethylaminomethyl-3-(4-methanesulfonylphenyl)-cyclopent-2-enyl ester,
5-dimethylaminomethyl-3-(4-methanesulfonylphenyl)-2-m-tolylcyclopent-2-enol,
and in each case corresponding salts, preferably hydrochlorides, and in each case corresponding solvates, preferably hydrates.

11. A method for producing substituted cyclopentene compounds according to claims 1 to 10, **characterised in that** at least one compound of the general formula II 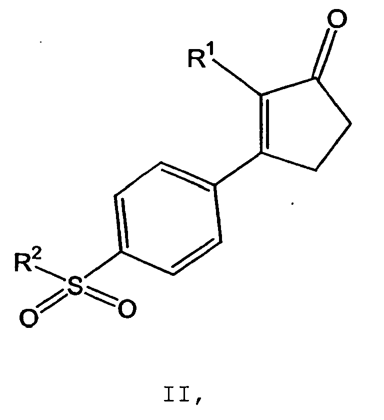 in which R¹ and R² have the meaning according to claims 1-10, is converted by reaction with formaldehyde and/or paraformaldehyde and at least one substituted amine compound of the general formula IIIa 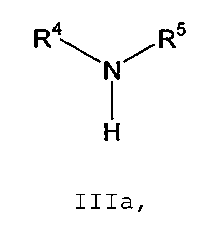 in which R⁴ and R⁵ have the meaning according to claims 1-10, or at least one substituted amine compound of the general formula IIIb, 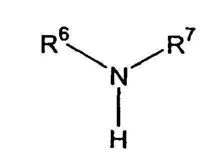 in which R⁶ and R⁷ have the meaning according to claims 1 to 10, or by reaction with at least one methyleneimmonium compound of the general formula IVa 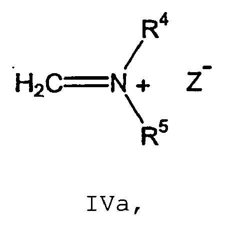 in which R⁴ and R⁵ have the meaning according to claims 1 to 10 and Z denotes a chlorine, bromine or iodine atom, or with at least one methyleneimmonium compound of the general formula IVb 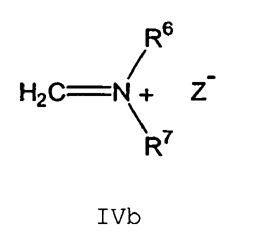 in which R⁶ and R⁷ have the meaning according to claims 1 to 10 and Z denotes a chlorine, bromine or iodine atom, into a compound of the above-stated general formula I according to claims 1 to 10, in which R¹ to R³ have the meaning according to claims 1 to 10, and A denotes a -(C=O) group, is optionally purified, optionally isolated, and
is optionally converted by reaction with at least one reducing agent into a compound of the general formula I according to claims 1 to 10, in which R¹ to R³ have the meaning according to claims 1 to 10, and A denotes a C(H) (R⁸) group and R⁸ denotes a hydroxy group, is optionally purified, optionally isolated,
and this compound is optionally converted by reaction with an esterification reagent into a compound of the general formula I, in which R¹ to R³ have the meaning according to claims 1 to 10, A denotes a C (H) (R⁸) group and R⁸ an -O(C=O)-R⁹ group, in which R⁹ has the meaning according to claims 1 to 10, and this compound is optionally purified and optionally isolated.

12. A medicament containing at least one cyclopentene compound according to claims 1 to 10 and optionally at least one further pharmacologically active active ingredient and optionally one or more physiologically acceptable auxiliary substances.

13. A medicament according to claim 12, **characterised in that** the further pharmacologically active active ingredient is selected from the group consisting of nonsteroidal antiinflammatory drugs (NSAID), triptans and ergot alkaloids.

14. A medicament according to claim 13, **characterised in that** the nonsteroidal antiinflammatory active ingredient is selected from the group consisting of acetylsalicylic acid, naproxen, diclofenac, ibuprofen, ketoprofen, piroxicam, diflunisal, indomethacin, tolmetin and celecoxib.

15. A medicament according to claim 13, **characterised in that** the triptan is selected from the group consisting of sumatriptan, eletriptan, rizatriptan, zolmitriptan and naratriptan.

16. A medicament according to claim 13, **characterised in that** ergotamine or dihydroergotamine is present as the ergot alkaloid.

17. A medicament according to one or more of claims 12-16 for CGRP receptor regulation, preferably for CGRP1 receptor regulation, for the treatment and/or prevention of disorders and/or diseases which are mediated by the CGRP receptor, preferably by the CGRP1 receptor, for the prevention and/or treatment of pain, preferably acute pain, chronic pain, chronic inflammatory pain or visceral pain, cluster headaches, neurovascular disorders, migraine, preferably of migraine with aura, migraine without aura, simple migraine, classical migraine or complicated migraine, inflammation, preferably pulmonary inflammation, asthma, arthritis, hypertonia, hypotonia, tachycardia, non-insulin-dependent diabetes mellitus, cardiovascular diseases, skin conditions and/or skin damage, preferably of skin damage caused by heat and/or radiation, particularly preferably of skin damage caused by sunburn, allergic rhinitis, diseases associated with overshooting vascular dilation, preferably shock or sepsis, menopausal hot flushes or opioid tolerance, preferably morphine tolerance.

18. Use of at least one compound according to claims 1 to 10 for producing a medicament for CGRP receptor regulation, preferably for CGRP1 receptor regulation.

19. Use of at least one compound according to claims 1 to 10 for producing a medicament for the treatment and/or prevention of disorders and/or diseases which are mediated by the CGRP receptor, preferably by the CGRP1 receptor.

20. Use according to claim 19 for the prevention and/or treatment of pain, preferably acute pain, chronic pain, chronic inflammatory pain or visceral pain.

21. Use according to claim 19 for the treatment and/or prevention of cluster headaches.

22. Use according to claim 19 for the prevention and/or treatment of neurovascular disorders.

23. Use according to claim 19 for the prevention and/or treatment of migraine, preferably of migraine with aura, migraine without aura, simple migraine, classical migraine or complicated migraine.

24. Use according to claim 19 for the prevention and/or treatment of inflammation, preferably pulmonary inflammation.

25. Use according to claim 19 for the prevention and/or treatment of asthma.

26. Use according to claim 19 for the prevention and/or treatment of arthritis.

27. Use according to claim 19 for the prevention and/or treatment of hypertonia.

28. Use according to claim 19 for the prevention and/or treatment of hypotonia.

29. Use according to claim 19 for the prevention and/or treatment of tachycardia.

30. Use according to claim 19 for the prevention and/or treatment of non-insulin-dependent diabetes mellitus.

31. Use according to claim 19 for the prevention and/or treatment of cardiovascular diseases.

32. Use according to claim 19 for the prevention and/or treatment of skin conditions and/or skin damage, preferably of skin damage caused by heat and/or radiation, particularly preferably of skin damage caused by sunburn.

33. Use according to claim 19 for the prevention and/or treatment of allergic rhinitis.

34. Use according to claim 19 for the prevention and/or treatment of diseases associated with overshooting vascular dilation, preferably shock or sepsis.

35. Use according to claim 19 for the prevention and/or treatment of menopausal hot flushes.

36. Use according to claim 19 for the reduction of opioid tolerance, preferably morphine tolerance.

## Revendications

1. Composés substitués de cyclopentène de formule générale I, 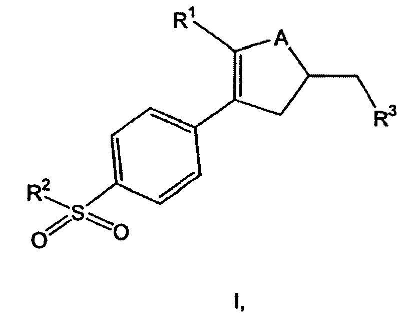 où
R¹ représente un radical phényle le cas échéant au moins monosubstitué, qui peut être condensé avec un système cyclique monocyclique le cas échéant au moins monosubstitué, saturé, insaturé ou aromatique, présentant le cas échéant au moins un hétéroatome comme élément de cycle,
R² représente un radical alkyle linéaire ou ramifié ou un groupe -NH₂,
R³ représente un groupe -NR⁴R⁵ ou un groupe -NR⁶R⁷,
R⁴ et R⁵ représentent, de manière identique ou différente, à chaque fois hydrogène ou un radical alkyle linéaire ou ramifié, à condition que les deux radicaux R⁴ et R⁵ ne signifient pas simultanément hydrogène,
R⁶ et R⁷ forment, ensemble avec l'atome d'azote formant un pont entre eux comme élément de cycle, un radical hétérocyclique saturé, le cas échéant au moins monosubstitué, présentant le cas échéant au moins un autre hétéroatome comme élément de cycle,
A représente un groupe C(=O) ou un groupe C(H)(R⁸),
R⁸ représente un groupe OH ou un groupe -O-(C=O)-R⁹,
R⁹ représente un radical alkyle linéaire ou ramifié,
le cas échéant à chaque fois sous forme de leurs stéréo-isomères purs, en particulier leurs énantiomères ou diastéréo-isomères, leurs racémates ou sous forme de mélanges de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

2. Composés selon la revendication 1, **caractérisés en ce que** R¹ représente un radical phényle le cas échéant au moins monosubstitué, qui peut être condensé avec un système monocyclique de 5 ou 6 chaînons, le cas échéant au moins monosubstitué, saturé, insaturé ou aromatique, présentant le cas échéant au moins un hétéroatome comme élément de cycle, de préférence un radical phényle, naphtyle ou benzo[1,3]-dioxole le cas échéant au moins monosubstitué.

3. Composés selon la revendication 1 ou 2, **caractérisés en ce que** R² représente un radical C₁₋₆-alkyle linéaire ou ramifié ou un groupe -NH₂, de préférence un radical C₁₋₄-alkyle linéaire ou ramifié ou un groupe NH₂, de manière particulièrement préférée un radical méthyle, éthyle ou un groupe NH₂, de manière tout particulièrement préférée un radical méthyle.

4. Composés selon l'une quelconque des revendications 1 à 3, **caractérisés en ce que** R⁴ et R⁵ représentent, de manière identique ou différente, à chaque fois hydrogène ou un radical C₁₋₆-alkyle linéaire ou ramifié, de préférence à chaque fois hydrogène ou un radical C₁₋₄-alkyle linéaire ou ramifié, de manière particulièrement préférée à chaque fois hydrogène, un radical méthyle ou éthyle, de manière tout particulièrement préférée tous les deux, simultanément, un radical méthyle.

5. Composés selon l'une quelconque des revendications 1 à 4, **caractérisés en ce que** R⁶ et R⁷ forment, ensemble avec l'atome d'azote formant un pont entre eux comme élément de cycle, un radical hétérocyclique de 5 à 8 chaînons, saturé, le cas échéant au moins monosubstitué, présentant le cas échéant au moins un autre hétéroatome comme élément de cycle, de préférence un cycle pyrrolidine, pipéridine, hexaméthylène-imine ou morpholine le cas échéant au moins monosubstitué.

6. Composés selon l'une quelconque des revendications 1 à 5, **caractérisés en ce que** A représente un groupe -C(=O).

7. Composés selon l'une quelconque des revendications 1 à 6, **caractérisés en ce que** R⁹ représente un radical C₁₋₆-alkyle linéaire ou ramifié, de préférence un radical C₁₋₄-alkyle linéaire ou ramifié, de manière particulièrement préférée un radical méthyle.

8. Composés selon l'une ou plusieurs des revendications 1 à 7, **caractérisés en ce que**
R¹ représente un radical phényle, naphtyle ou benzo[1,3]dioxolyle, qui est à chaque fois non substitué ou substitué par 1, 2, 3, 4 ou 5 substituants choisis indépendamment l'un de l'autre dans le groupe constitué par -F ; -Cl; -Br, -I ; -CF₃ ; -OH ; méthyle ; éthyle ; n-propyle ; iso-propyle ; n-butyle ; sec-butyle ; iso-butyle ; tert-butyle ; méthoxy ; éthoxy ; n-propyloxy ; iso-propyloxy ; n-butyloxy ; sec-butyloxy ; iso-butyloxy ; tert-butyloxy ; -SO₂-méthyle ; -SO₂-éthyle ; -SO₂-n-propyle ; -SO₂-isopropyle ; -SO₂-n-butyle ; -SO₂-sec-butyle ; -SO₂-isobutyle ; -SO₂-tert-butyle et -SO₂-NH₂,
R² représente un groupe méthyle ou un groupe -NH₂,
R³ représente un groupe -NR⁴R⁵,
R⁴ et R⁵ représentent à chaque fois un groupe méthyle ;
A représente un groupe C(=O) ou un groupe C(H)(R⁸),
R⁸ représente un groupe OH ou un groupe -O-(C=O)-R⁹,
R⁹ représente un groupe méthyle ou éthyle ;
le cas échéant à chaque fois sous forme de leurs stéréo-isomères purs, en particulier leurs énantiomères ou diastéréo-isomères, leurs racémates ou sous forme de mélanges de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

9. Composés selon la revendication 8, **caractérisés en ce que** R¹ représente un radical qui est choisi dans le groupe constitué par
2-fluorophényle ; 3-fluorophényle ; 4-fluorophényle ; 2-chlorophényle ; 3-chlorophényle ; 4-chlorophényle ; 2-bromophényle ; 3-bromophényle ; 4-bromophényle ; 3,5-difluorophényle ; 3,5-dichlorophényle ; 3,5-dibromophényle ; 2-trifluorométhylphényle ; 3-trifluorométhylphényle ; 4-trifluorométhylphényle ; 4-fluoro-3-méthylphényle ; o-toluyle ; m-toluyle ; p-toluyle ; 2-éthylphényle ; 3-éthylphényle ; 4-éthylphényle ; 2-iso-propylphényle ; 3-iso-propylphényle ; 4-iso-propylphényle ; 2-tert-butylphényle ; 3-tert-butylphényle ; 4-tert-butylphényle ; 3,4-diméthylphényle ; 2-hydroxyphényle ; 3-hydroxyphényle ; 4-hydroxyphényle ; 2-méthoxyphényle ; 3-méthoxyphényle ; 4-méthoxyphényle ; 2-éthoxyphényle ; 3-éthoxyphényle ; 4-éthoxyphényle ; 2-méthanesulfonylphényle ; 3-méthanesulfonylphényle ; 4-méthanesulfonylphényle ; benzo[1,3]dioxo-5-yle ; napht-1-yle et napht-2-yle.

10. Composés selon l'une ou plusieurs des revendications 1 à 9 choisis dans le groupe constitué par
- la 2-(3,5-difluorophényl)-5-diméthylaminométhyl-3-(4-méthanesulfonylphényl)-cyclopent-2-énone,
- la 5-diméthylaminométhyl-3-(4-méthanesulfonylphényl)-2-m-toluylcyclopent-2-énone,
- la 5-diméthylaminométhyl-2-(3-hydroxyphényl)-3-(4-méthanesulfonylphényl)-cyclopent-2-énone,
- la 5-diméthylaminométhyl-2-(4-fluorophényl)-3-(4-méthanesulfonylphényl)-cyclopent-2-énone,
- la 5-diméthylaminométhyl-2-(3-fluorophényl)-3-(4-méthanesulfonylphényl)-cyclopent-2-énone,
- la 2-(3-chlorophényl)-5-diméthylaminométhyl-3-(4-méthanesulfonylphényl)-cyclopent-2-énone,
- la 5-diméthylaminométhyl-3-(4-méthanesulfonylphényl)-2-p-toluylcyclopent-2-énone,
- la 5-diméthylaminométhyl-2-(4-hydroxyphényl)-3-(4-méthanesulfonylphényl)-cyclopent-2-énone,
- la 5-diméthylaminométhyl-3-(4-méthanesulfonylphényl)-2-(3-trifluorométhylphényl)-cyclopent-2-énone,
- la 5-diméthylaminométhyl-3-(4-méthanesulfonylphényl)-2-(4-méthoxyphényl)-cyclopent-2-énone,
- la 2-benzène[1,3]dioxol-5-yl-5-diméthylaminométhyl-3-(4-méthanesulfonylphényl)-cyclopent-2-énone,
- la 2-(3,5-dichlorophényl)-5-diméthylaminométhyl-3-(4-méthanesulfonylphényl)-cyclopent-2-énone,
- la 5-diméthylaminométhyl-3-(4-méthanesulfonylphényl)-2-(3-méthoxyphényl)-cyclopent-2-énone,
- la 5-diméthylaminométhyl-2-(4-fluoro-3-méthylphényl)-3-(4-méthanesulfonylphényl)-cyclopent-2-énone,
- la 2-(4-tert-butylphényl)-5-diméthylaminométhyl-3-(4-méthanesulfonylphényl)-cyclopent-2-énone,
- la 5-diméthylaminométhyl-2-(3-éthoxyphényl)-3-(4-méthanesulfonylphényl)-cyclopent-2-énone,
- la 5-diméthylaminométhyl-2,3-bis-(4-méthanesulfonylphényl)-cyclopent-2-énone,
- la 5-diméthylaminométhyl-3-(4-méthanesulfonylphényl)-2-napht-2-yl-cyclopent-2-énone,
- la 5-diméthylaminométhyl-2-(3,4-diméthylphényl)-3-(4-méthanesulfonylphényl)-cyclopent-2-énone
- la 5-diméthylaminométhyl-2-(3-isopropylphényl)-3-(4-méthanesulfonylphényl)-cyclopent-2-énone,
- le 2-(4-tert-butylphényl)-5-diméthylaminométhyl-3-(4-méthanesulfonylphényl)-cyclopent-2-énol,
- l'ester 2-(4-tert-butylphényl)-5-diméthylaminométhyl-3-(4-méthanesulfonylphényl)-cyclopent-2-énylique de l'acide acétique,
- le 5-diméthylaminométhyl-3-(4-méthanesulfonylphényl)-2m-toluylcyclopent-2-énol,
et à chaque fois les sels correspondants, de préférence des chlorhydrates et à chaque fois les solvates correspondants, de préférence les hydrates.

11. Procédé pour la préparation de composés substitués de cyclopentène selon les revendications 1 à 10, **caractérisé en ce qu'**on transforme au moins un composé de formule générale II 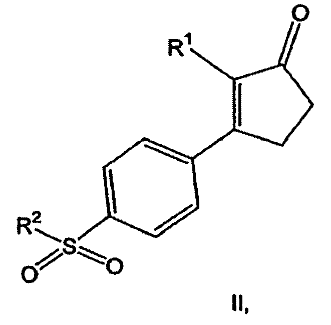 où R¹ et R² ont la signification selon les revendications 1-10, par transformation avec du formaldéhyde et/ou du paraformaldéhyde et au moins un composé amine substitué de formule générale IIIa 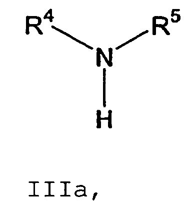 où R⁴ et R⁵ ont la signification selon les revendications 1 à 10, ou au moins un composé amine substitué de formule générale IIIb, 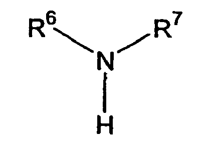 où R⁶ et R⁷ ont la signification selon les revendications 1 à 10, ou par transformation avec au moins un composé de méthylène-immonium de formule générale IVa 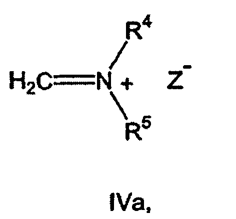 où R⁴ et R⁵ ont la signification selon les revendications 1 à 10 et Z représente un atome de chlore, de brome ou d'iode, ou par transformation avec au moins un composé de méthylène-immonium de formule générale IVb 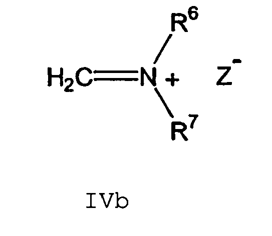 où R⁶ et R⁷ ont la signification selon les revendications 1 à 10 et Z représente un atome de chlore, de brome ou d'iode, en un composé de formule générale I selon les revendications 1 à 10, où R¹ à R³ ont la signification selon les revendications 1 à 10 et A représente un groupe -(C=O)-, on le purifie le cas échéant, on l'isole le cas échéant et
on le transforme le cas échéant par transformation avec au moins un agent de réduction en un composé de formule générale I selon les revendications 1 à 10, où R¹ à R³ ont la signification selon les revendications 1 à 10 et A représente un groupe -C (H) (R⁸) - et R⁸ représente un groupe hydroxy, on le purifie le cas échéant, on l'isole le cas échéant,
et on transforme celui-ci le cas échéant par transformation avec au moins un réactif d'estérification en un composé de formule générale I, où R¹ à R³ ont la signification selon les revendications 1 à 10, A représente un groupe C (H) (R⁸) et R⁸ représente un groupe -O(C=O)-R⁹, où R⁹ a la signification selon les revendications 1 à 10, on le purifie le cas échéant et on l'isole le cas échéant.

12. Médicament contenant au moins un composé de cyclopentène selon les revendications 1 à 10 ainsi que le cas échéant au moins une autre substance pharmacologiquement active et le cas échéant un ou plusieurs adjuvants physiologiquement acceptables.

13. Médicament selon la revendication 12, **caractérisé en ce que** l'autre substance pharmacologiquement active est choisie dans le groupe constitué par les substances actives anti-inflammatoires non stéroïdiennes (NSAID), les triptans et les alcaloïdes d'ergot de seigle.

14. Médicament selon la revendication 13,
**caractérisé en ce que** la substance active anti-inflammatoire non stéroïdienne est choisie dans le groupe constitué par l'acide acétylsalicylique, la Naproxène, le Diclofénac, l'Ibuprofène, le Kétoprofène, le Piroxicam, le Diflunisal, l'Indométhacine, la Tolmétine et le Célécoxib.

15. Médicament selon la revendication 13, **caractérisé en ce que** le triptan est choisi dans le groupe constitué par le sumatriptan, l'életriptan, le rizatriptan, le zolmitriptan et le naratriptan.

16. Médicament selon la revendication 13, **caractérisé en ce que** l'alcaloïde d'ergot de seigle est l'ergotamine ou la dihydroergotamine.

17. Médicament selon l'une ou plusieurs des revendications 12-16 pour la régulation du récepteur de CGRP, de préférence pour la régulation du récepteur de CGRP1, pour le traitement et/ou la prophylaxie de troubles et/ou de maladies qui sont favorisées par le récepteur de CGRP, de préférence par le récepteur de CGRP-1, pour la prophylaxie et/ou le traitement de douleurs, de préférence les douleurs aiguës, les douleurs chroniques, les douleurs chroniques inflammatoires ou les douleurs viscérales, les céphalées de tension, les troubles neurovasculaires, les migraines, de préférence les migraines avec aura, les migraines sans aura, les migraines simples, les migraines classiques ou les migraines compliquées, les inflammations, de préférence les inflammations pulmonaires, l'asthme, l'arthrite, l'hypertonie, l'hypotonie, la tachycardie, le diabète sucré non-insulino-dépendant, les maladies cardiovasculaires, les maladies de la peau et/ou les lésions de la peau, de préférence les lésions de la peau provoquées thermiquement et/ou par un rayonnement, de manière particulièrement préférée les lésions de la peau provoquées par les brûlures par le soleil, la rhinite allergique, les maladies qui vont de pair avec un élargissement excessif des vaisseaux, de préférence un choc ou une septicémie, les bouffées de chaleur ménopausiques ou la tolérance aux opioïdes, de préférence la tolérance à la morphine.

18. Utilisation d'au moins un composé selon les revendications 1 à 10 pour la préparation d'un médicament destiné à la régulation du récepteur de CGRP, de préférence du récepteur de CGRP-1.

19. Utilisation d'au moins un composé selon les revendications 1 à 10 pour la préparation d'un médicament destiné au traitement et/ou à la prophylaxie de troubles et/ou de maladies qui sont favorisées par le récepteur de CGRP, de préférence par le récepteur de CGRP-1.

20. Utilisation selon la revendication 19 pour la prophylaxie et/ou le traitement de douleurs, de préférence de douleurs aiguës, de douleurs chroniques, de douleurs chroniques inflammatoires ou de douleurs viscérales.

21. Utilisation selon la revendication 19 pour le traitement et/ou la prophylaxie de céphalées de tension.

22. Utilisation selon la revendication 19 pour la prophylaxie et/ou le traitement de troubles neurovasculaires.

23. Utilisation selon la revendication 19 pour la prophylaxie et/ou le traitement de migraines, de préférence de migraines avec aura, de migraines sans aura, de migraines simples, de migraines classiques ou de migraines compliquées.

24. Utilisation selon la revendication 19 pour la prophylaxie et/ou le traitement d'inflammations, de préférence d'inflammations pulmonaires.

25. Utilisation selon la revendication 19 pour la prophylaxie et/ou le traitement de l'asthme.

26. Utilisation selon la revendication 19 pour la prophylaxie et/ou le traitement de l'arthrite.

27. Utilisation selon la revendication 19 pour la prophylaxie et/ou le traitement de l'hypertonie.

28. Utilisation selon la revendication 19 pour la prophylaxie et/ou le traitement de l'hypotonie.

29. Utilisation selon la revendication 19 pour la prophylaxie et/ou le traitement de la tachycardie.

30. Utilisation selon la revendication 19 pour la prophylaxie et/ou le traitement du diabète sucré non insulinodépendant.

31. Utilisation selon la revendication 19 pour la prophylaxie et/ou le traitement de maladies cardiovasculaires.

32. Utilisation selon la revendication 19 pour la prophylaxie et/ou le traitement de maladies de la peau et/ou de lésions de la peau, de préférence de lésions de la peau provoquées thermiquement et/ou par un rayonnement, de manière particulièrement préférée de lésions de la peau provoquées par les brûlures par le soleil.

33. Utilisation selon la revendication 19 pour la prophylaxie et/ou le traitement de la rhinite allergique.

34. Utilisation selon la revendication 19 pour la prophylaxie et/ou le traitement de maladies qui vont de pair avec un élargissement excessif des vaisseaux, de préférence un choc ou une septicémie.

35. Utilisation selon la revendication 19 pour la prophylaxie et/ou le traitement de bouffées de chaleur ménopausiques.

36. Utilisation selon la revendication 19 pour la diminution de la tolérance aux opioïdes, de préférence la tolérance à la morphine.
